# EUROPEAN PATENT SPECIFICATION

(11) **EP 3 784 284 B1**
(45) Date of publication and mention of the grant of the patent: **02.08.2023**
(21) Application number: 19718759.4
(22) Date of filing: 25.04.2019
(51) Int. Cl.: A61K 47/10, A61K 9/127, A61K 48/00, A61K 47/69

(54) **LIPID-BASED FORMULATIONS FOR THE DELIVERY OF RNA**
LIPID-BASIERTE ZUSAMMENSETZUNGEN ZUR VERABREICHUNG VON RNA
FORMULATIONS A BASE DE LIPIDES POUR LA DELIVRANCE D'ARN

(30) Priority: 25.04.2018 EP 18169325; 14.08.2018 EP 18189010
(43) Date of publication of application: 03.03.2021
(73) Proprietor: Ethris GmbH, 82152 Planegg (DE)
(72) Inventor: DOHMEN, Christian, 82061 Neuried (DE); MYKHAILYK, Olga, 88175 Scheidegg (DE)
(74) Representative: Vossius & Partner Patentanwälte Rechtsanwälte mbB
(86) International application number: PCT/EP2019/060644
(87) International publication number: WO 2019/207060

(56) References cited:
- EP-B1- 1 954 252
- WO-A1-2018/089540
- ANDRIES O. ET AL.: "Comparison of the gene transfer efficiency of mRNA/GL67 and pDNA/GL67 complexes in respiratory cells", MOLECULAR PHARMACEUTICS, no. 9, 2012, pages 2136-2145, XP055282775,
- JULIA CHRISTINA KASPER ET AL: "Development of a lyophilized plasmid/LPEI polyplex formulation with long-term stability A step closer from promising technology to application", JOURNAL OF CONTROLLED RELEASE, ELSEVIER, AMSTERDAM, NL, vol. 151, no. 3, 4 January 2011 (2011-01-04), pages 246-255, XP028215426, ISSN: 0168-3659, DOI: 10.1016/J.JCONREL.2011.01.003 [retrieved on 2011-01-09]

## Description

The present invention relates to lipid based compositions which can be used for the efficient administration of an RNA to a subject.

Various lipid based formulations are known in the art which can act as vectors for nucleic acids so as to allow them to be delivered to the body of a patient, e.g. A.C. Silva et al., Current Drug Metabolism, 16, 2015, 3-16, and the literature referred to therein. A formulation which has been successfully tested in clinical studies for pDNA delivery via inhalation is GL67A (E. Alton et al., Efficacy and Mechanism Evaluation, Vol. 3, Issue 5, July 2016). The formulation GL67A contains, together with pDNA as a therapeutically active nucleic acid, GL67 (also known as Genzyme Lipid 67, a cationically derivatized cholesterol), 1,2-dioleoyl-*sn*-glycero-3-phosphoethanolamine (DOPE) and 1,2-dimyristoyl-*sn*-glycero-3-phosphoethanolamine-PEG5000 (DMPE-PEG5000).

However, the formulation shows a limited stability (t ½: 6h), such that it had to be prepared shortly before its application. Moreover, the lipid formulation was found to be ineffective for the transfection of RNA *in vivo* (O. Andries et al., Molecular Pharmaceutics 2012, 9, 2136-2145). In view of these problems, a need remains for formulations which can act as effective vectors for the delivery of RNA *in vivo* while providing a beneficial toxicological profile.

In the context of the present invention, it has been found that the presence of 1,2-propanediol (also referred to as propylene glycol) leads to a significant increase of the transfection efficiency of lipid based formulations of the type used in GL67A which contain RNA as a nucleic acid.

Thus, in accordance with a first aspect, the invention provides a composition comprising
(i) particles contained in a liquid phase, wherein the particles comprise RNA and a lipid composition, and wherein the lipid composition comprises:
   (i-a) a cholesterol derivative of formula (I) or a salt thereof: 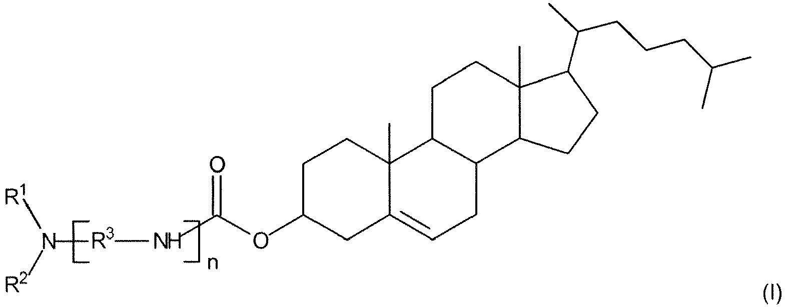 wherein
      - n: is 0 or 1, preferably 0,
      - R¹: is a group -(CH₂)_{q}-NH₂ or a group -(CH₂)ᵣ-NH-(CH₂)ₛ-NH₂, wherein q, r and s are independently an integer of 2 to 6,
      - R²: is a group -(CH₂)ₜ-NH₂ or a group -(CH₂)ᵤ-NH-(CH₂)_{w}-NH₂, wherein t, u and w are independently an integer of 2 to 6,
      - R³: is a linear alkanediyl group having 1 to 4 carbon atoms;
   (i-b) a phosphoglyceride of formula (II) or a salt thereof: 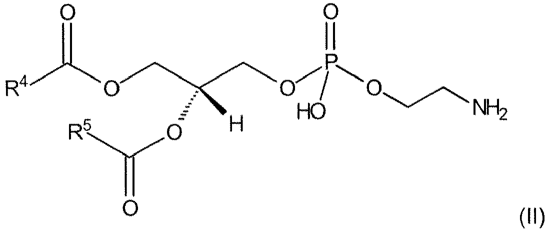 wherein
      - R⁴: is a linear alkyl group having 10 to 20 carbon atoms or a linear alkenyl group having 1 to 3 double bonds and 10 to 20 carbon atoms;
      - R⁵: is a linear alkyl group having 10 to 20 carbon atoms or a linear alkenyl group having 1 to 3 double bonds and 10 to 20 carbon atoms;
      and
   (i-c) a pegylated phosphoglyceride of formula (III) or a salt thereof: 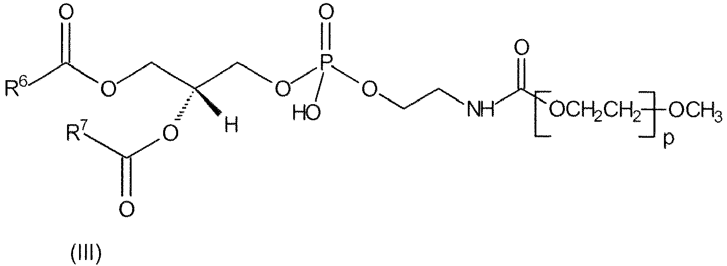 wherein
      - p: is an integer of 5 to 200, preferably 10 to 170 and most preferably 10 to 140
      - R⁶: is a linear alkyl group having 10 to 20 carbon atoms or a linear alkenyl group having 1 to 3 double bonds and 10 to 20 carbon atoms;
      - R⁷: is a linear alkyl group having 10 to 20 carbon atoms or a linear alkenyl group having 1 to 3 double bonds and 10 to 20 carbon atoms;
      and
(ii) 1,2-propanediol.

A second aspect of the invention relates to a process for the preparation of a composition in accordance with the first aspect described above, said process comprising the steps of
a) dissolving and mixing the components of the lipid composition in an organic solvent, followed by the lyophilization of the lipid composition;
b) rehydrating the lyophilized lipid composition via addition of water;
c) combining the rehydrated lipid composition with an aqueous solution of the RNA to allow particles comprising RNA and the lipid composition to be formed which are contained in a liquid phase;
d) adding 1,2-propanediol.

The addition of the 1,2-propanediol may be conveniently accomplished e.g. by adding it together with water in step b), by adding it to the rehydrated lipid composition following step b), by adding it together with the aqueous solution of the RNA in step c), or by adding it to the liquid phase following step c), wherein the particles comprising RNA and the lipid composition are contained. It will be appreciated that the addition can be carried out in one step or in multiple steps, e.g. at different stages of the process.
The 1,2-propanediol may enhance the stability of particle formulations of RNA during freezing, e.g. when such a particle formulation is frozen for storage or handling. In other words, the 1,2-propanediol may also function as a cryoprotectant for particles comprising RNA and the lipid composition.

In the following, a detailed description will be provided of the invention and of its aspects discussed above. It will be appreciated in this context that these aspects are closely interrelated. Thus, it will be understood that the detailed information which is provided with regard to features of one aspect will apply also for other aspects which rely on this feature, unless indicated otherwise.

### RNA

The composition and the solid composition in accordance with the invention comprise ribonucleic acid (RNA), more preferably single stranded RNA, and most preferred is mRNA.

As regards RNA, in principle any type of RNA can be employed in the context of the present invention. In a preferred embodiment the RNA is a single-stranded RNA. The term "single-stranded RNA" means a single consecutive chain of ribonucleotides in contrast to RNA molecules in which two or more separate chains form a double-stranded molecule due to hybridization of the separate chains. The term "single-stranded RNA" does not exclude that the single-stranded molecule forms in itself double-stranded structures such as secondary (e.g. loops and stem-loops) or tertiary structures.

The term "RNA" covers RNA which codes for an amino acid sequence as well as RNA which does not code for an amino acid sequence. It has been suggested that more than 80 % of the genome contains functional DNA elements that do not code for proteins. These noncoding sequences include regulatory DNA elements (binding sites for transcription factors, regulators and coregulators etc.) and sequences that code for transcripts that are never translated into proteins. These transcripts, which are encoded by the genome and transcribed into RNA but do not get translated into proteins, are called noncoding RNAs (ncRNAs). Thus, in one embodiment the RNA is a noncoding RNA. Preferably, the noncoding RNA is a single-stranded molecule. Studies demonstrate that ncRNAs are critical players in gene regulation, maintenance of genomic integrity, cell differentiation, and development, and they are misregulated in various human diseases. There are different types of ncRNAs: short (20-50 nt), medium (50-200 nt), and long (>200 nt) ncRNAs. Short ncRNA includes microRNA (miRNA), small interfering RNA (siRNA), piwi-interacting RNA (piRNA), and transcription initiating RNA (tiRNA). Examples of medium ncRNAs are small nuclear RNAs (snRNAs), small nucleolar RNAs (snoRNAs), transfer RNAs (tRNAs), transcription start-site-associated RNAs (TSSaRNAs), promoter-associated small RNAs (PASRs), and promoter upstream transcripts (PROMPTs). Long noncoding RNAs (IncRNA) include long-intergenic noncoding RNA (IincRNA), antisense-IncRNA, intronic IncRNA, transcribed ultra-conserved RNAs (T-UCRs), and others (Bhan A, Mandal SS, ChemMedChem. 2014 Mar 26. doi: 10.1002/cmdc.201300534). Of the above-mentioned non-coding RNAs only siRNA is double-stranded. Thus, since in a preferred embodiment the noncoding RNA is single-stranded, it is preferred that the noncoding RNA is not siRNA. In another embodiment the RNA is a coding RNA, i.e. an RNA which codes for an amino acid sequence. Such RNA molecules are also referred to as mRNA (messenger RNA) and are single-stranded RNA molecules. The RNA may be made by synthetic chemical and enzymatic methodology known to one of ordinary skill in the art, or by the use of recombinant technology, or may be isolated from natural sources, or by a combination thereof.

Messenger RNAs (mRNA) are copolymers which are built up of nucleoside phosphate building blocks mainly with adenosine, cytidine, uridine and guanosine as nucleosides, which as intermediate carriers bring the genetic information from the DNA in the cell nucleus into the cytoplasm, where it is translated into proteins. They are thus suitable as alternatives for gene expression.

In the context of the present invention, mRNA should be understood to mean any polyribonucleotide molecule which, if it comes into the cell, is suitable for the expression of a protein or fragment thereof or is translatable to a protein or fragment thereof. The term "protein" here encompasses any kind of amino acid sequence, i.e. chains of two or more amino acids which are each linked via peptide bonds and also includes peptides and fusion proteins.

The mRNA contains a ribonucleotide sequence which encodes a protein or fragment thereof whose function in the cell or in the vicinity of the cell is needed or beneficial, e.g. a protein the lack or defective form of which is a trigger for a disease or an illness, the provision of which can moderate or prevent a disease or an illness, or a protein which can promote a process which is beneficial for the body, in a cell or its vicinity. The mRNA may contain the sequence for the complete protein or a functional variant thereof. Further, the ribonucleotide sequence can encode a protein which acts as a factor, inducer, regulator, stimulator or enzyme, or a functional fragment thereof, where this protein is one whose function is necessary in order to remedy a disorder, in particular a metabolic disorder or in order to initiate processes in vivo such as the formation of new blood vessels, tissues, etc. Here, functional variant is understood to mean a fragment which in the cell can undertake the function of the protein whose function in the cell is needed or the lack or defective form whereof is pathogenic. In addition, the mRNA may also have further functional regions and/or 3' or 5' noncoding regions. The 3' and/or 5' noncoding regions can be the regions naturally flanking the protein-encoding sequence or artificial sequences which contribute to the stabilization of the RNA. Those skilled in the art can determine the sequences suitable for this in each case by routine experiments.

In a preferred embodiment, the mRNA contains a 5'-cap (five-prime-cap; cap-0) consisting of a m7GpppG connected to the mRNA via a 5' to 5' triphosphate linkage, an additional methyl group onto the penultimate nucleotide from the 5'-end of the mRNA (Cap-1, Anti-Reverse Cap Analog (ARCA)) and/or an internal ribosome entry site (IRES) and/or a polyA-tail at the 3'-end, in particular, in order to improve translation. The mRNA can have further regions promoting translation such as, for example, cap-2 structures or histone stem-loop structures.

As noted above, unless indicated otherwise in a specific context, the term mRNA as used herein encompasses modified mRNA, i.e. the mRNA may be modified mRNA.

In another embodiment, the mRNA contains labeled nucleic acids (preferably, nucleotides and/or ribonucleotides) such as, for example isotope and/or fluorescence labelled nucleotides Labelled mRNA molecules play for example an important role in studying the intracellular conformation of RNA and DNA molecules.

In a preferred embodiment, the RNA, preferably the mRNA, is a molecule which contains modified nucleotides/ribonucleotides. In addition to the four classical ribonucleotides, namely, adenosine, guanosine, cytidine and uridine, there exist numerous analogs of each of these nucleobases. Sometimes throughout and in the literature, these analogs, or RNA/mRNA molecules that include one or more of these analogs, are referred to as modified in terms of the present invention (e.g., modified nucleotides or modified ribonucleotides). Some analogs differ from the above canonical nucleobases, but yet can exist in nature. Other analogs are non-naturally occurring. Either type of analog is contemplated. Preferred modifications are set out in the following table (Table 1):

| Name | Base modification (5-position) | Sugar modification (2'-position) | Naturally in mRNA |
|---|---|---|---|
| Uridine | | | |
| 5-methyluridine 5'-triphosphate (m5U) | CH₃ | - | no |

| 5-iodouridine 5'-triphosphate (I5U) | I | - | no |
|---|---|---|---|
| 5-bromouridine 5'-triphosphate (Br5U) | Br | - | no |

| 2-thiouridine 5'-triphosphate (S4U) | S (in 2 position) | - | no |
|---|---|---|---|
| 4-thiouridine 5'-triphosphate (S2U) | S (in 4 position) | - | no |
| 2'-methyl-2'-deoxyuridine 5'-triphosphate (U2'm) | - | CH₃ | yes |
| 2'-amino-2'-deoxyuridine 5'-triphosphate (U2'NH2) | - | NH₂ | no |
| 2'-azido-2'-deoxyuridine 5'-triphosphate (U2'N3) | - | N₃ | no |
| 2'-fluoro-2'-deoxyuridine 5'-triphosphate (U2'F) | - | F | no |
| Pseudouridine (ψ) | - | - | yes |
| N1-methyl-pseudouridine (N1mΨ) | - | - | no |

| Cytidine | | | |
|---|---|---|---|
| 5-methylcytidine 5'-triphosphate (m5C) | CH₃ | - | yes |
| 5-iodocytidine 5'-triphosphate (I5U) | I | - | no |
| 5-bromocytidine 5'-triphosphate (Br5U) | Br | - | no |
| 2-thiocytidine 5'-triphosphate (S2C) | S (in 2 position) | - | no |
| 2'-methyl-2'-deoxycytidine 5'-triphosphate (C2'm) | - | CH₃ | yes |
| 2'-amino-2'-deoxycytidine 5'-triphosphate (C2'NH2) | - | NH₂ | no |
| 2'-azido-2'-deoxycytidine 5'-triphosphate (C2'N3) | - | N₃ | no |
| 2'-fluoro-2'-deoxycytidine 5'-triphosphate (C2'F) | - | F | no |

| Adenosine | | | |
|---|---|---|---|
| N6-methyladenosine 5'-triphosphate (m6A) | CH₃ (in 6 position) | - | yes |
| N1-methyladenosine 5'-triphosphate (m1A) | CH₃ (in 1 position) | - | no |
| 2'-0-methyladenosine 5'-triphosphate (A2'm) | - | CH₃ | yes |
| 2'-amino-2'-deoxyadenosine 5'-triphosphate (A2'NH2) | - | NH₂ | no |
| 2'-azido-2'-deoxyadenosine 5'-triphosphate (A2'N3) | - | N₃ | no |
| 2'-fluoro-2'-deoxyadenosine 5'-triphosphate (A2'F) | - | F | no |

| Guanosine | | | |
|---|---|---|---|
| N1-methylguanosine 5'-triphosphate (m1G) | CH₃ (in 1 position) | - | no |
| 2'-O-methylguanosine 5'-triphosphate (G2'm) | - | CH₃ | yes |
| 2'-amino-2'-deoxyguanosine 5'-triphosphate (G2'NH2) | - | NH₂ | no |
| 2'-azido-2'-deoxyguanosine 5'-triphosphate (G2'N3) | - | N₃ | no |
| 2'-fluoro-2'-deoxyguanosine 5'-triphosphate (G2'F) | - | F | no |

For the RNA, preferably the mRNA, according to the invention, either all uridine nucleotides and cytidine nucleotides can each be modified in the same form or else a mixture of modified nucleotides can be used for each. The modified nucleotides can have naturally or not naturally occurring modifications. A mixture of various modified nucleotides can be used. Thus for example one part of the modified nucleotides can have natural modifications, while another part has modifications not occurring naturally or a mixture of naturally occurring modified and/or not naturally occurring modified nucleotides can be used. Also, a part of the modified nucleotides can have a base modification and another part a sugar modification. In the same way, it is possible that all modifications are base modifications or all modifications are sugar modifications or any suitable mixture thereof. By variation of the modifications, the stability and/or duration of action of the RNA, preferably the mRNA, according to the invention can be selectively adjusted.

In one embodiment of the invention, at least two different modifications are used for one type of nucleotide, where one type of the modified nucleotides has a functional group via which further groups can be attached. Nucleotides with different functional groups can also be used, in order to provide binding sites for the attachment of different groups. Thus for example a part of the modified nucleotides can bear an azido group, an amino group, a hydroxy group, a thiol group or some other reactive group which is suitable for reaction under predefined conditions. The functional group can also be such that it can under certain conditions activate a naturally present group capable of binding, so that molecules with functions can be coupled. Nucleotides which are modified so that they provide binding sites can also be introduced as adenosine or guanosine modifications. The selection of the particular suitable modifications and the selection of the binding sites to be made available depend on what groups are to be introduced and with what frequency these are to be present. Thus the content of the nucleotides provided with functional and/or activating groups depends on how high the content of groups to be coupled is to be and can easily be determined by those skilled in the art. As a rule, the content of nucleotides modified with functional and/or activating groups, if present, is 1 to 25% of the modified nucleotides. Those skilled in the art can if necessary determine the most suitable groups in each case and the optimal content thereof by routine experiments.

In a preferred embodiment, the RNA, preferably the mRNA according to the invention is characterized in that modified uridines are selected from 2-thiouridine, 5-methyluridine, pseudouridine, 5-methyluridine 5'-triphosphate (m5U), 5-idouridine 5'-triphosphate (I5U), 4-thiouridine 5'-triphosphate (S4U), 5-bromouridine 5'-triphosphate (Br5U), 2'-methyl-2'-deoxyuridine 5'-triphosphate (U2'm), 2'-amino-2'-deoxyuridine 5'-triphosphate (U2'NH2), 2'-azido-2'-deoxyuridine 5'-triphosphate (U2'N3) and 2'-fluoro-2'-deoxyuridine 5'-triphosphate (U2'F).

In another preferred embodiment, the RNA, preferably the mRNA according to the invention is characterized in that modified cytidines are selected from 5-methylcytidine, 3-methylcytidine, 2-thio-cytidine, 2'-methyl-2'-deoxycytidine 5'-triphosphate (C2'm), 2'-amino-2'-deoxycytidine 5'-triphosphate (C2'NH2), 2'-fluoro-2'-deoxycytidine 5'-triphosphate (C2'F), 5-iodocytidine 5'-triphosphate (I5U), 5-bromocytidine 5'-triphosphate (Br5U) and 2'-azido-2'-deoxycytidine 5'-triphosphate (C2'N3).

In a preferred embodiment the mRNA is an mRNA which contains a combination of modified and unmodified nucleotides. Preferably, it is an mRNA containing a combination of modified and unmodified nucleotides as described in WO2011/012316. The mRNA described therein is reported to show an increased stability and diminished immunogenicity. In a preferred embodiment, in such a modified mRNA 5 to 50% of the cytidine nucleotides and 5 to 50% of the uridine nucleotides are modified. The adenosine- and guanosine-containing nucleotides can be unmodified. The adenosine and guanosine nucleotides can be unmodified or partially modified, and they are preferably present in unmodified form. Preferably 10 to 35% of the cytidine and uridine nucleotides are modified and particularly preferably the content of the modified cytidine nucleotides lies in a range from 7.5 to 25% and the content of the modified uridine nucleotides in a range from 7.5 to 25%. It has been found that in fact a relatively low content, e.g. only 10% each, of modified cytidine and uridine nucleotides can achieve the desired properties. It is particularly preferred that the modified cytidine nucleotides are 5-methylcytidin residues and the modified uridine nucleotides are 2-thiouridin residues. Most preferably, the content of modified cytidine nucleotides and the content of the modified uridine nucleotides is 25%, respectively.

In certain other embodiments, in such a modified RNA, preferably mRNA, 5 to 50% of the cytidines are analogs of C and 5 to 50% of the uridines are analogs of U. In certain embodiments, in such a modified RNA, preferably mRNA, 5 to 40% of the cytidines are analogs of C and 5 to 40% of the uridines are analogs of U. In certain embodiments, in such a modified RNA, preferably mRNA, 5 to 30% of the cytidines are analogs of C and 5 to 30% of the uridines are analogs of U. In certain embodiments, in such a modified RNA, preferably mRNA, 10 to 30% of the cytidines are analogs of C and 10 to 30% of the uridines are analogs of U. In certain embodiments, in such a modified RNA, preferably mRNA, 5 to 20% of the cytidines are analogs of C and 5 to 20% of the uridines are analogs of U. In certain embodiments, in such a modified RNA, preferably mRNA 5 to 10% of the cytidine nucleotides and 5 to 10% of the uridine nucleotides are modified. In certain embodiments, in such a modified RNA, preferably mRNA, 25% of the cytidine nucleotides and 25% of the uridine nucleotides are modified. In certain embodiments, the adenosine- and guanosine-containing nucleotides can be unmodified. In certain embodiments, the adenosine and guanosine nucleotides can be unmodified or partially modified, and they are preferably present in unmodified form.

As noted above, in certain embodiments, analogs of U refers to a single type of analog of U. In certain embodiments, analogs of U refers to two or more types of analogs of U. In certain embodiments, analogs of C refers to a single type of analog of C. in certain embodiments, analogs of C refers to two or more types of analogs of C.

in certain embodiments, the percentage of cytidines in an RNA, preferably an mRNA that are analogs of cytidine is not the same as the percentage of uridines in the RNA preferably in the mRNA that are analogs of uridine. In certain embodiments, the percentage of analogs of cytidine is lower than the percentage of analogs of uridine. As noted above, this may be in the presence or the absence of analogs of adenosine and guanosine but, in certain embodiments, is in the absence of analogs of adenosine and analogs of guanosine. In certain embodiments, RNA, preferably the mRNA of the disclosure comprises less than 15%, less than 10%, less than 5% or less than 2% analogs of adenosine, analogs of guanosine or both.

In certain embodiments, an RNA, preferably an mRNA of the present invention comprises analogs of cytidine and analogs of uridine, and 5 to 20% of the cytidines are analogs of cytidine and 25 to 45% of the uridines are analogs of uridine. In other words, the RNA, preferably the mRNA comprises modified and unmodified cytidines and modified and unmodified uridines, and 5 to 20% of the cytidines comprise analogs of cytidine while 25 to 45% of the uridines comprise analogs of uridine. In other embodiments, the RNA, preferably the mRNA comprises 5 to 10% analogs of cytidine and 30 to 40% analogs of uridine, such as 7-9% analogs of cytidine, such as about 7, 7.5 or 8% and, such as 32-38% analogs of uridine, such as about 33, 34, 35, 36%.

In certain embodiments, any of the analogs of uridine and analogs of cytidine described herein may be used, optionally excluding pseudouridine. In certain embodiments, the analog of cytidine comprises or consists of (e.g., in the case of consists of, it is the single analog type used) 5-iodocytidine and the analog of uridine comprises or consists of (e.g., in the case of consists of, it is the single analog type used) 5-iodouridine.

In certain embodiments of any of the foregoing, the percentage of analogs of a given nucleotide refers to input percentage (e.g., the percentage of analogs in a starting reaction, such as a starting in vitro transcription reaction). In certain embodiments of any of the foregoing, the percentage of analogs of a given nucleotide refers to output (e.g., the percentage in a synthesized or transcribed compound). Both options are equally contemplated.

The RNA, preferably the mRNA molecules of the present invention may be produced recombinantly in *in vivo* systems by methods known to a person skilled in the art.

Alternatively, the modified RNA, preferably the mRNA molecules of the present invention may be produced in an *in vitro* system using, for example, an *in vitro* transcription system which is known to the person skilled in the art. An *in vitro* transcription system capable of producing RNA, preferably mRNA requires an input mixture of modified and unmodified nucleoside triphosphates to produce modified RNA, preferably mRNA molecules with the desired properties of the present invention. In certain embodiments, 5 to 50% of the cytidines are analogs of cytidine in such an input mixture and 5 to 50% of the uridines are analogs of uridine in such an input mixture. In certain embodiments, 5 to 40% of the cytidines are analogs of cytidine in such an input mixture and 5 to 40% of the uridines are analogs of uridine in such an input mixture. In certain embodiments, 5 to 30% of the cytidines are analogs of cytidine in such a mixture and 5 to 30% of the uridines are analogs of uridine in such an input mixture. In certain embodiments, 5 to 30% of the cytidines are analogs of cytidine in such mixture and 10 to 30% of the uridines are analogs of uridine in such mixture. In certain embodiments, 5 to 20% of the cytidines are analogs of cytidine in such an input mixture and 5 to 20% of the uridines are analogs of uridine in such an input mixture. In certain embodiments, 5 to 10% of the cytidines are analogs of cytidine in such an input mixture and 5 to 10% of the uridines are analogs of uridine in such an input mixture. In certain embodiments, 25% of the cytidines are analogs of cytidine in such an input mixture and 25% of the uridines are analogs of uridine in such an input mixture. In certain embodiments, the input mixture does not comprise analogs of adenosine and/or guanosine. In other embodiments, optionally, the input mixture comprises one or more analogs of adenosine and/or guanosine (or none of either or both).

In certain embodiments, the percentage of cytidines in an input mixture that are analogs of cytidine is not the same as the percentage of uridines in an input mixture that are analogs of uridine. In certain embodiments, the percentage of analogs of cytidine in an input mixture is lower than the percentage of analogs of uridine in an input mixture. As noted above, this may be in the presence or the absence of analogs of adenosine and guanosine in the input mixture but, in certain embodiments, is in the absence of analogs of adenosine and analogs of guanosine in the input mixture.

In certain embodiments, an input mixture of nucleotides for an *in vitro* transcription system that produces a RNA, preferably mRNA of the present invention comprises analogs of cytidine and analogs of uridine, and 5 to 20% of the cytidines of the input mixture are analogs of cytidine and 25 to 45% of the uridines of the input mixture are analogs of uridine. In other words, the input mixture comprises modified and unmodified cytidines and modified and unmodified uridines, and 5 to 20% of the cytidines of the input mixture comprise analogs of cytidine while 25 to 45% of the uridines of the input mixture comprise analogs of uridine. In other embodiments, the input mixture comprises 5 to 10% analogs of cytidine and 30 to 40% analogs of uridine, such as 7-9% analogs of cytidine, such as 7, 7.5 or 8% and, such as 32-38% analogs of uridine, such as 33, 34, 35, 36%.

In certain embodiments, any of the analogs of uridine and analogs of cytidine described herein may be used, optionally excluding pseudouridine. In certain embodiments, the analog of cytidine comprises or consists of (e.g., it is the single C analog type used) 5-iodocytidine and the analog of uridine comprises or consists of (e.g., it is the single U analog type used) 5-iodouridine.

Exemplary analogs are described in the tables above. It should be understood that for modified polyribonucleotides encoding the desired polypeptide (module (a)), the analogs and level of modification is, unless indicated otherwise, considered across the entire polyribonucleotide encoding the desired polypeptide (module (a)), including 5' and 3' untranslated regions (e.g., the level of modification is based on input ratios of analogs in an in vitro transcription reaction such that analogs may be incorporated at positions that are transcribed).

Furthermore, the modified RNA, preferably mRNA molecules may be chemically synthesized, e.g., by conventional chemical synthesis on an automated nucleotide sequence synthesizer using a solid-phase support and standard techniques or by chemical synthesis of the respective DNA sequences and subsequent *in vitro* or *in vivo* transcription of the same.

In another preferred embodiment, the mRNA may be combined with target binding sites, targeting sequences and/or with micro-RNA binding sites, in order to allow activity of the desired mRNA only in the relevant cells. In a further preferred embodiment, the RNA can be combined with micro-RNAs or shRNAs downstream of the 3' polyA tail.

In general, therapeutic effects can be achieved by the interaction of the ribonucleic acid with cellular molecules and organelles. Such interaction alone may for example activate the innate immune system, as is the case for certain CpG oligonucleotides and sequences designed to specifically interact with toll-like and other extra- or intracellular receptors. Furthermore, the uptake or introduction of ribonucleic acids (preferably mRNAs) in cells can be intended to lead to the expression of nucleotide sequences such as genes comprised in the ribonucleic acid (preferably the mRNA), can be intended for the downregulation, silencing or knockdown of endogenous gene expression as a consequence of the intracellular presence of an introduced exogenous nucleic acid, or can be intended for the modification of endogenous nucleic acid sequences such as repair, excision, insertion or exchange of selected bases or of whole stretches of endogenous nucleic acid sequences, or can be intended for interference with virtually any cellular process as a consequence of the intracellular presence and interaction of an introduced exogenous ribonucleic acid (preferably an mRNA). Overexpression of introduced exogenous ribonucleic acids (preferably mRNAs) may be intended to compensate or complement endogenous gene expression, in particular in cases where an endogenous gene is defective or silent, leading to no, insufficient or a defective or a dysfunctional product of gene expression such as is the case with many metabolic and hereditary diseases like cystic fibrosis, hemophilia or muscular dystrophy to name a few. Overexpression of introduced exogenous ribonucleic acids (preferably mRNAs) may also be intended to have the product of the expression interact or interfere with any endogenous cellular process such as the regulation of gene expression, signal transduction and other cellular processes. The overexpression of introduced exogenous ribonucleic acids (preferably mRNAs) may also be intended to give rise to an immune response in context of the organism in which a transfected or transduced cell resides or is made to reside. Examples are the genetic modification of antigen-presenting cells such as dendritic cells in order to have them present an antigen for vaccination purposes. Other examples are the overexpression of cytokines in tumors in order to elicit a tumor-specific immune response. Furthermore, the overexpression of introduced exogenous ribonucleic acids (preferably mRNAs) may also be intended to generate in vivo or ex vivo transiently genetically modified cells for cellular therapies such as modified T-cells or precursor or stem or other cells for regenerative medicine.

Downregulation, silencing or knockdown of endogenous gene expression for therapeutic purposes can for example be achieved by RNA interference (RNAi), with ribozymes, antisense oligonucleotides, tRNAs, long double-stranded RNA where such downregulation can be sequence-specific or unspecific and can also lead to cell death as is the case when long double-stranded RNAs are introduced into cells. Downregulation, silencing or knockdown of endogenous or pre-existing gene expression can be useful in the treatment of acquired, hereditary or spontaneously incurring diseases including viral infections and cancer. It can also be envisaged that the introduction of nucleic acids into cells can be practiced as a preventive measure in order to prevent, for example, viral infection or neoplasias. Downregulation, silencing or knockdown of endogenous gene expression can be exerted on the transcriptional level and on the translational level. Multiple mechanisms are known to the one skilled in the art and include for example epigenetic modifications, changes in chromatin structure, selective binding of transcription factors by the introduced nucleic acid, hybridization of the introduced nucleic acid to complementary sequences in genomic DNA, mRNA or other RNA species by base pairing including unconventional base pairing mechanisms such as triple helix formation. Similarly, gene repair, base or sequence changes can be achieved at the genomic level and at the mRNA level including exon skipping. Base or sequence changes can for example be achieved by RNA-guided site-specific DNA cleavage, by cut and paste mechanisms exploiting trans-splicing, trans-splicing ribozymes, chimeraplasts, splicosome-mediated RNA trans-splicing, or by exploiting group II or retargeted introns, or by exploiting insertional mutagenesis mediated by viruses or exploiting targeted genomic insertion using prokaryotic, eukaryotic or viral integrase systems. As nucleic acids are the carriers of the building plans of living systems and as they participate in many cellular processes in a direct and indirect manner, in theory any cellular process can be influenced by the introduction of nucleic acids into cells from outside. Notably, this introduction can be carried out directly in vivo and ex vivo in cell or organ culture followed by transplantation of thus modified organs or cells into a recipient. The particles for use in the context of the present invention with nucleic acids as therapeutically active agent may be useful for all purposes described above.

As mentioned above, the RNA, preferably the mRNA, may contain a ribonucleotide sequence which encodes a protein or fragment thereof whose function in the cell or in the vicinity of the cell is needed or beneficial, e.g. a protein the lack or defective form of which is a trigger for a disease or an illness, the provision of which can moderate or prevent a disease or an illness, or a protein which can promote a process which is beneficial for the body, in a cell or its vicinity.

Indeed, in recent years, RNA (in particular, mRNA) has become increasingly relevant as a new drug entity. As opposed to DNA-based gene therapeutics, mRNA does not need to be transported into the nucleus but is directly translated into protein in the cytoplasm (J Control Release, 2011, 150:238-247, and Eur J Pharm Biopharm, 2009, 71:484-489).

Moreover, numerous genetic disorders, caused by the mutation of a single gene are known and candidates for RNA, preferably the mRNA, therapeutic approaches. Disorders caused by single-gene mutations, like cystic fibrosis, hemophilia and many others, can be dominant or recessive with respect to the likelihood that a certain trait will appear in the offspring. While a dominant allele manifests a phenotype in individuals who have only one copy of the allele, for a recessive allele the individual must have two copies, one from each parent to become manifest. In contrast, polygenic disorders are caused by two or more genes and the manifestation of the respective disease is often fluent and associated to environmental factors. Examples for polygenic disorders are hypertension, elevated cholesterol level, cancer, neurodegenerative disorders, mental illness and others. Also in these cases therapeutic RNA, preferably the mRNA, representing one or more of these genes may be beneficial to those patients. Furthermore, a genetic disorder must not have been passed down from the parents' genes, but can also be caused by new mutations. Also in these cases therapeutic RNA, preferably the mRNA, representing the correct gene sequence may be beneficial to the patients.

An online catalog with presently 22,993 entries of Human Genes and Genetic Disorders together with their respective genes and a description of their phenotypes are available at the ONIM (Online Mendelian Inheritance in Man) webpage (http://onim.org); sequences of each are available from the Uniprot database (http://www.uniprot.org). As non-limiting examples, the following **Table 2** lists some congenital diseases, and the corresponding gene(s). Due to the high degree of interaction of cellular signaling pathways, the mutation of a certain gene causes a multiply of pathogenic symptoms, of which only a characteristic one is listed in **Table 2.**

in some embodiments of the present invention, the therapeutic protein which is encoded by the RNA, preferably the mRNA, of the present invention is chosen from the cellular proteins listed in **Table 2.** Thus, the RNA, preferably the mRNA, molecule of the invention may encode a therapeutic cellular protein, wherein the encoded therapeutic protein is one listed in **Table 2** or a homolog thereof.

In another embodiment of the present invention, the therapeutic protein which is encoded by the RNA, preferably the mRNA, of the present invention is chosen from the secreted proteins listed in **Table 2.** Thus, the RNA, preferably the mRNA, of the present invention may encode a therapeutic fusion protein, wherein the encoded therapeutic protein or a homolog thereof is one listed in **Table 2** and the second protein is a signal peptide that allows the secretion of the therapeutic protein. A signal peptide is a short, typically 5-30 amino acids long, amino acids sequence present at the N-terminus of said therapeutic protein and that leads the fusion protein towards the cell's secretory pathway via certain organelles (i.e. the endoplasmic reticulum, the golgi-apparatus or the endosomes). Thus, such fusion protein is secreted from the cell or from a cellular organelle or inserted into a cellular membrane (e.g. multi-spanning trans- membrane proteins) at a cellular compartment or at the cell's surface.

Thus, in preferred embodiments of the present invention the RNA, preferably the mRNA, of the present invention may encode, but is not limited to, the following proteins of the genes that cause, predispose or protect from diseases. Non-limiting examples of such disorders that may be treated (or prevented) include those wherein said polypeptide, protein or peptide is selected from the group consisting of the ones as outlined in the following **Table 2.**

in some embodiments, the encoding sequence of the RNA, preferably the mRNA, of the present invention may be transcribed and translated into a partial or full length protein comprising cellular activity at a level equal to or greater than that of the native protein. In some embodiments, the RNA, preferably the mRNA, of the present invention encodes a therapeutically or pharmaceutically active polypeptide, protein or peptide having a therapeutic or preventive effect, wherein said polypeptide, protein or peptide is selected from the group consisting of the ones as outlined in the following **Table 2.** The RNA, preferably the mRNA, more specifically the encoding sequence thereof, may be used to express a partial or full length protein with cellular activity at a level equal to or less than that of the native protein. This may allow the treatment of diseases for which the administration of an RNA molecule can be indicated.

**Table 2: Non-limiting examples of human genes and genetic disorders**

| **Disease** | | **Pathology** | **Gene, heredity** |
|---|---|---|---|
| | | | |
| | **Blood diseases** | | |
| Fanconi Anemia | | Anemia and neutropenia, evidence that a DNA repair mechanism is affected | FANCA, autosomal recessive |
| Hemophilia-A | | Abnormal bleeding | Coagulation Factor VIII, X-chromosomal recessive |
| Hemophilia-B | | Abnormal bleeding | Coagulation Factor IX, X-chromosomal recessive |
| Hereditary Spherocytosis (various types) | | spherical-shaped erythrocytes (spherocytes) | Ankyrin (ANK1) |
| Paroxysmal nocturnal hemoglobinuria | | Anemia and presence of blood in the urine | PIG-A, X-chromosomal |
| Porphyria cutanea tarda | | Overproduction of heme, iron overload | Uroporphyrinogen decarboxylase (UROD), autosomal recessive |
| Severe combined immune deficiency (SCID) | | Due to impaired DNA synthesis severe immune deficiency in humoral and cellular immunity | Adenosine deaminase, autosomal recessive, IL-2R-y, JAK3, (IL-7R-α, RAG1/2, Artemis, CD3δ, CD3ε |
| Sickle-cell anemia | | Abnormal hemoglobin (HbS) | β-Hemoglobin (HB), autosomal recessive |
| Thalassemia (α- and β form) | | Lack of α- or β hemoglobin resulting in anemia | Deletion of HBA1 and/or HBA2, |
| Von Willebrand disease (three types known, Type-III is most severe) | | Abnormal bleeding, hemorrhage similar to hemophilia A and B | Autosomal dominant and recessive forms |
| | | | |

| | **Cancer** | | |
|---|---|---|---|
| Malignant melanoma | | P16 mutation leads to uncontrolled proliferation of fibroblasts | Cyclie dependant kinase inhibitor 2 (CDKN2) |
| Neurofibromatosis (2 types) | | Benign tumors on auditory nerves leads to deafness | NF1, NF2, autosomal dominant |
| | | | |

| | **Deafness (Ear)** | | |
|---|---|---|---|
| Deafness | | Hearing loss | Deafness-1A (DFNB1), autosomal recessive |
| Pendred syndrome | | Hearing loss | Pendrin (PDS), autosomal recessive |
| | | | |

| | **Heart** | | |
|---|---|---|---|
| Ataxia telangiectasia | | DNA damage repair disturbed, | ATM, |
| Atherosclerosis | | increase of blood cholesterol | apoE, |
| LQT Syndrome (Long QT) | | Potassium channel defect | LQT1 and other genes |
| Von-Hippel Lindau Syndrome | | Abnormal growth of blood vessels, can lead to cancer | VHL, autosomal dominant |
| William's Beuren Syndrome | | Deletion of elastin results in vascular defects, supravalvular aortic stenosis | Deletion of elastin and LIM kinase genes |
| | | | |

| | **Metabolic disorders and glycogen storage diseases** | | |
|---|---|---|---|
| Adrenoleukodystrophy | | Disturbed fatty acid transport and metabolism | ABCD1, X-chromosomal |
| Alkaptonuria | | Nitrogen metabolism defect, Urine turns dark when exposed to oxygen | Homogentisic Oxidase, autosomal recessive |
| Diabetes type I | | Disturbed insulin | IDDM1, IDDM2, GCK, ... |
| | | production | |
| Galactosemia | | disorder of galactose metabolism | Galactose-1-phosphate uridyltransferase gene (GALT), autosomal recessive |
| Gauche disease | | Disturbance of fat metabolism | Glucocerebrosidase |
| Glucose Galactosidase Malabsorption | | Disturbed glucose and galactose transport out of the intestinal lumen resulting in diarrhea | SGLT1, autosomal recessive |
| Glycogen storage disease Type I, Von-Gierke's disease | | Accumulation of glucose in liver and kidney | Glucose-6-Phosphatase, autosomal recessive |
| Glycogen storage disease Type II, Pompe's disease | | Accumulation of glycogen in liver, heart, skeletal muscle, cardiomegaly | α-1-Glucosidase, autosomal recessive |
| Glycogen storage disease Type III, Cori's disease | | Accumulation of glycogen in liver, heart, skeletal muscle, hepatoomegaly | Debranching enzyme, autosomal recessive |
| Glycogen storage disease Type V, McArdle's disease | | Cannot untilize glycogen in muscle cells | Muscle phosphorylase, autosomal recessive |
| Glucose-6-Phosphate Dehydrogenase | | Inability to maintain glutathione leads to hemolytic anemia | G6PD, X-chromosomal recessive |
| Hereditary Hemochromatosis (4 types) | | Excess of iron in the body (esp. liver) due to excessive iron absorption in the gut | Hemochromatosis (HFE) |
| Homocystinuria | | Nitrogen metabolism defect | Cystathione synthetase defect, autosomal recessive |
| Lesh Nyhan Syndrome | | Accumulation of uric acid leading to gout, ureate stones and muscle loss | HPRT1, X-chromosomal |
| Maple Syrup Urine Disease | | Amino acid metabolism defect leads to the accumulation of α-Ketoacides and death in the first months if untreated | Branched-chain-alpha-dehydrogenase (BCKDH) |
| Menkes' Syndrome | | Reduced ability to absorb copper, leads to death in infancy if untreated | ATP7A , X-chromosomal recessive |
| Obesity | | Elevated body weight | Polygenic, elevated leptin levels may play a role |
| Phenylketonuria | | Inability to break down Phenylalanine into tyrosine leads to mental retardation | Phenylalanine hydroxylase (PAH), autosomal recessive |
| Tangier disease | | reduced levels of plasma high density lipoproteins | ATP-binding cassette-1 gene (ABCA1) |
| Zellweger Syndrome (leads to death in infants) | | High levels of iron and copper in the blood | PXR1 (receptor on the surface of peroxisomes) |
| Wilsons Disease | | Copper accumulation in brain and liver | ATP7B (P-type ATPase), autosomal recessive |
| | | | |

| | **Musculoskeletal system** | | |
|---|---|---|---|
| Achondroplasis | | Short stature with a large head due to slow proliferation of chondrocytes | Fibroblast growth factor receptor 3 (FGF3R), |
| Charcot-Marie-Tooth Syndrome and its more severe form Dejerine-Sottas Syndrome | | Degeneration of the muscles in limbs | Different forms caused by different gene mutations, autosomal recessive and X-chromosomal |
| Cockayne syndrome (2 types) | | Premature aging and short stature, loss of "on the fly" DNA repair | group 8 excision repair cross-complementing protein (ERCC8) |
| Chondroectodermal dysplasia | | Malformation of bones and polydactyly | EVC, autosomal recessive |
| Diastrophic dysplasia (DTD) | | Malformed hands, sulfate transporter defect | DTDST gene |
| Duchenne muscular dystrophy | | Enlargement of muscle tissue with subsequent loss of function | DMD, X-chromosomal recessive |
| Fibrodysplasia Ossificans Progressiva | | Heterotopic bone formation | NOG, BMP, Autosomal dominant |
| Friedreich's ataxia | | Heart enlargement and progressive loss of muscular coordination | Frataxin, autosomal recessive |
| Hypophosphatasia | | Production of an abnormal version of alkaline phosphatase affecting the mineralization process | ALPL, autosomal recessive |
| Marfan Syndrome | | Connective tissue disorder due fibrillin deficiency | Fibrillin 1 (FBN), autosomal dominant |
| Myotonic dystrophy (onset during young adulthood) | | Protein kinase defect in skeletal muscle cells | Dystrophia myotonica protein kinase (DMPK), autosomal dominant |
| Osteogenesis imperfect (various types) | | Defect in type-I collagen formation leads to multiple fractures after birth | COL1A1, COL1A2 |
| Prader-Willi Syndrome | | Decreased muscle tone and mental retardation | SNRPN (small ribinucleoprotein N) deleted due to a deletion on chromosome 15 |
| | | | |

| | **Neurons and Brain** | | |
|---|---|---|---|
| Alzheimer disease | | Increased amyloid production, progressive inability to remember facts | Polygenic, PS1, PS2, ... |
| Amyotrophic lateral sclerosis (ALS) (various forms) | | Progressive degeneration of motor neuron cells (defect in elimination superoxide | Superoxide dismutase 1 (SOD1), various genes involved |
| | | radicals) | |
| Angelman syndrome | | Mental retardation with inadequate laughing | Genomic imprinting on chromosome 15 |
| Pyruvat dehydrogenase | | Neurological defects if untreated | Pyruvat dehydrogenase, autosomal recessive |
| Refsum disease | | Accumulation of phytanic acid leads to peripheral neuropathy | Phytanoyl-CoA hydroxylase (PHYH), autosomal recessive |
| Rett's syndrome | | Mental retardation with arrested development between 6 and 18 months of age | Methyl-CpG-binding protein-2 (MECP2), X-chromosomal dominant |
| Tay-Sachs disease (various forms of severity) | | Disturbed break down of GM2 ganglioside leads to neurological damage | HEXA (β-hexosaminidas A), autosomal recessive |
| LaFora Disease | | Aggressive form of epilepsy | EPM2A, autosomal recessive |
| Essential tremor (variable forms) | | Uncontrollable shaking | ETM1, ETM2, autosomal dominant |
| Fragile X syndrome | | Lack of FMR1 RNA binding protein, mental retardation | FMR1 gene is not expressed due to an CGG amplification in the 5'UTR region |
| Huntington's disease | | Progressive dementia with onset in adulthood | HTT (huntingtin), autosomal dominant |
| | | | |

| | **Intestine** | | |
|---|---|---|---|
| Bartter's syndrome (3 types) | | Renal disease | Kidney chloride channel B gene (CLCNKB), autosomal recessive |
| Polycystic kidney disease (2 types) | | renal disease | PDK1, PDK2, autosomal dominant, there is also a autosomal recessive form known (ARPKD) |
| | | | |

| | **Lung** | | |
|---|---|---|---|
| Alpha-1-antitrypsin | | Defect alveoli due to uncontrolled release of elastase | SERPINA1, autosomal codominant |
| Asthma | | Chronic inflammatory disorder of the airways | Polygenic |
| Cystic fibrosis | | Excessively viscous mucous due to defective Cl⁻ ion transport | CFTR (cystic fibrosis conductance transmembrane regulator), autosomal recessive |
| Surfactant metabolism dysfunction (various types) | | Newborns are of normal body weight, but all fail to inflate | ATP-binding cassette transporter (ABCA3) |
| Primary cliliary dyskinesia | | Excessively viscous mucous due to defective/missing cilia function | DNAI1, CCNO, CCDC40 among others |
| | | | |

| | **Lysosomal storage diseases** | | |
|---|---|---|---|
| Fabry's disease | | Beyond others, skin lesions due to the accumulation of | α-Galactosidase A, X-chromosomal recessive |
| | | ceramide trihexoside | |
| Gaucher's Disease | | Accumulation of glucocerebrosides (gangliosides, sphingolipids) | Glucocerebrosidase, autosomal recessive, |
| Type-I: adult form (normal lifespan under treatment) | | | |
| Type-II: infantile form (death before age 1) | | | |
| Type-III: juvenile form (onset in early childhood, less severe than Type-II) | | | |
| Hunter's Syndrome | | Accumulation of mucopolysaccharides | L-iduronosulfat sulfatase, X-chromosomal recessive |
| Hurler's Syndrome (death by age of 10) | | Accumulation of mucopolysaccharides | α-L-iduronidase, autosomal recessive |
| Niemann-Pick Disease (three distinct forms A, B, C) | | Defect in releasing Cholesterol from lysosomes, accumulation of Sphingomyelin | Sphingomyelinase, autosomal recessive |
| Tay-Sachs disease (death by age of 4) | | Accumulation of G_{M2} ganglioside in neuronal cells | Hexosaminidase A, autosomal recessive |
| | | | |

| | **Skin** | | |
|---|---|---|---|
| Albinism | | Nitrogen metabolism defect | Tyrosinase deficiency, autosomal recessive |
| Albinism, oculocutaneous, type II | | Reduced biosynthesis of melanin pigment | OCA2, autosomal recessive |
| Ehlers-Danlos Syndrome (various types) | | Diaphragmatic hernia. common , retinal detachment | Various defects in collagen synthesis |
| Epidermolysis bullosa (various types including EB simplex, Junctional EB, Dystrophic EB and Kindler syndrome) | | Defects in maintenance of keratinocyte structural stability or adhesion of the keratinocyte to the underlying dermis | Epidermolysis bullosa macular type (EBM), Epidermolysis bullosa 3 progressiva (EBR3), Epidermolysis bullosa 4 pseudojunctual (EBR4), Desmoplakin (DSP), Plakophilin-1 (PKP1), kreatin (KRT5, KRT14), plectin (PLEC), ITGA6, integrin subunit (ITGB4), laminin subunits (LAMA3, LAMP3, LAMB3, LAMC2), collagen (COL17A1, COL7A1 (autosomal dominant), FERMT1, autosomal recessive |
| Hartnup's disease | | Defect in tryptophan uptake in the gastrointestinal tract, light-sensitive skin | SLC6A19, autosomal recessive |
| Hereditary Hemorrhagic Telangiectasia, Osler-Weber-Rendu Syndrome | | Telangiectasia of the skin and mucous membranes | Endoglin (ENG), autosomal dominant |
| Hypercholesterolemia, | | elevation of serum | Low-density lipoprotein |
| familial | | cholesterol bound to low density lipoprotein, accumulation in skin and arteriosclerosis | receptor (LDLR), apolipoprotein B (APOB), autosomal dominant |
| Xeroderma pigmentosa | | skin defect and melanoma due to UV exposure | DNA repair defect, autosomal recessive |
| Male pattern baldness | | Disturbed conversion of testosterone into dihydrotestosterone in the skin | 5-α-reductase |
| | | | |

| | **Genetic liver diseases** | | |
|---|---|---|---|
| Amino acid metabolism disorders | | Disruptions in the multistep process that breaks down the amino acid tyrosine and phenylalanine | FAH, TAT, HPD, autosomal recessive |
| Beta-thalassemia intermedia | | Shortage of mature red blood cells | HBB, autosomal recessive |
| Crigler-Najjar syndrome | | Deficiency in glucuronidation in which bilirubin gets dissolvable in water | UGT1A1, autosomal recessive |
| Fatty acid oxidation disorders | | Deficiency in processing of long-chain fatty acids and very long-chain fatty acids resulting in lethargy and hypoglycemia | HADHA, ACADVL autosomal recessive |
| Fructose metabolism disorders | | Impaired gluconeogenesis causing hypoglycemia | FBP1, ALDOB, autosomal recessive |
| Galactosemia | | Deficiency in processing galactose | GALT, GALK1, GALE, autosomal recessive |
| Glycogen storage diseases | | Disturbed breackdown of glucose 6-phosphate and glycogen leads to accumulation of glycogen as well as abnormal glycogen molecules causing cell damage | G6PC, SLC37A4, AGL, GBE1, autosomal recessive |
| Heme biosynthesis disorder | | Decrease of uroporphyrinogen decarboxylase resulting in accumulation of compounds called porphyrins causing toxic levels in liver | UROD autosomal dominant, ALAS2 X-limked dominant, ALAD autosomal recessive |
| Lipid metabolism (transport) disorders | | Shortage of functional protein, which prevents movement of cholesterol and other lipids, leading to their accumulation in cells | NPC1, NPC2 autosomal recessive, LDLR, autosomal dominant |
| Metal metabolism disorders | | Disorders in the storage and transport of iron and copper resulting in accumulation in tissues and organs | ATP7B, HAMP, HFE, HFE2, autosomal recessive |
| Organic acid disorders (Acidurias/Acidemias) | | Disrupted break down of several protein building blocks (amino acids), certain lipids, and cholesterol | BCKDHA, BCKDHB, and DBT, PCCA and PCCB, MUT, MMAA, MMAB, MMADHC, MCEE, IVD, MCCC1 or MCCC2, autosomal recessive |
| Primary hyperoxaluria type 1 | | Disrupted breakdown of glyoxylate leading to renal damage | AGXT, GRHPR, autosomal recessive |
| Progressive familial intrahepatic cholestasis | | Buildup of bile acids in liver cells causing liver damage | ATP8B1, autosomal recessive |
| Thrombocyte activity disorder | | Lack of enzyme activity disrupts the usual balance between bleeding and clotting | ADAMTS13, autosomal recessive |
| Urea cycle disorders | | Disorder of the urea cycle which causes a form of hyperammonemia | OTC (X-linked disorder), CPS1, ASS1 and SLC25A13, ASL, autosomal recessive |

The above **Table 2** shows examples of genes in which a defect leads to a disease which can be treated with the RNA, preferably the mRNA, of the present invention wherein RNA, preferably the mRNA, of the present invention comprises a ribonucleotide sequence which encodes an intact version of the protein or a functional fragment thereof of the above disclosed defective gene. In particularly preferred embodiments, hereditary diseases can be mentioned which for example affect the lungs, such as SPB (surfactant protein B) deficiency, ABCA3 deficiency, cystic fibrosis and α1-antitrypsin deficiency, or which affect plasma proteins (e.g. congenital hemochromatosis (hepcidin deficiency), thrompotic thrombocytopenic purpura (TPP, ADAMTS 13 deficiency) and cause clotting defects (e.g. haemophilia a and b) and complement defects (e.g. protein C deficiency), immune defects such as for example SCID (caused my mutations in different genes such as: RAG1, RAG2, JAK3, IL7R, CD45, CD3δ, CD3ε) or by deficiencies due to lack of adenosine desaminase for example (ADA-SCID), septic granulomatosis (e.g. caused by mutations of the gp-91-phox gene, the p47-phox gene, the p67-phox gene or the p33-phox gene) and storage diseases like Gaucher's disease, Fabry's disease, Krabbe's disease, MPS I, MPS II (Hunter syndrome), MPS VI, Glycogen storage disease type II or muccopolysacchaidoses.

Other disorders for which the RNA, preferably the mRNA, of the present invention can be useful include disorders such as SMN1-related spinal muscular atrophy (SMA); amyotrophic lateral sclerosis (ALS); GALT-related galactosemia; Cystic Fibrosis (CF); SLC3A1-related disorders including cystinuria; COL4A5-related disorders including Alport syndrome; galactocerebrosidase deficiencies; X-linked adrenoleukodystrophy and adrenomyeloneuropathy; Friedreich's ataxia; Pelizaeus-Merzbacher disease; TSC1 and TSC2-related tuberous sclerosis; Sanfilippo B syndrome (MPS IIIB); CTNS-related cystinosis; the FMR1-related disorders which include Fragile X syndrome, Fragile X-Associated Tremor/Ataxia Syndrome and Fragile X Premature Ovarian Failure Syndrome; Prader-Willi syndrome; hereditary hemorrhagic telangiectasia (AT); Niemann-Pick disease Type C1; the neuronal ceroid lipofuscinoses-related diseases including Juvenile Neuronal Ceroid Lipofuscinosis (JNCL), Juvenile Batten disease, Santavuori-Haltia disease, Jansky-Bielschowsky disease, and PTT-1 and TPP1 deficiencies; EIF2B1, EIF2B2, EIF2B3, EIF2B4 and EIF2B5-related childhood ataxia with central nervous system hypomyelination/vanishing white matter; CACNA1A and CACNB4-related Episodic Ataxia Type 2; the MECP2-related disorders including Classic Rett Syndrome, MECP2-related Severe Neonatal Encephalopathy and PPM-X Syndrome; CDKL5-related Atypical Rett Syndrome; Kennedy's disease (SBMA); Notch-3 related cerebral autosomal dominant arteriopathy with subcortical infarcts and leukoencephalopathy (CADASIL); SCN1A and SCN1B-related seizure disorders; the Polymerase G-related disorders which include Alpers-Huttenlocher syndrome, POLG-related sensory ataxic neuropathy, dysarthria, and ophthalmoparesis, and autosomal dominant and recessive progressive external ophthalmoplegia with mitochondrial DNA deletions; X-Linked adrenal hypoplasia; X-linked agammaglobulinemia; Fabry disease; and Wilson's disease.

In all these diseases, a protein, e.g. an enzyme, is defective, which can be treated by treatment with the RNA, preferably the mRNA, encoding any of the above proteins of the present invention, which makes the protein encoded by the defective gene or a functional fragment thereof available. Transcript replacement therapies/enzyme replacement therapies do not affect the underlying genetic defect, but increase the concentration of the enzyme in which the patient is deficient. As an example, in Pompe's disease, the transcript replacement therapy/enzyme replacement therapy replaces the deficient Lysosomal enzyme acid alpha-glucosidase (GAA).

Thus, non-limiting examples of proteins which can be encoded by the mRNA of the present invention are erythropoietin (EPO), growth hormone (somatotropin, hGH), cystic fibrosis transmembrane conductance regulator (CFTR), growth factors such as GM-SCF, G-CSF, MPS, protein C, hepcidin, ABCA3 and surfactant protein B. Further examples of diseases which can be treated with the RNA according to the invention are hemophilia A/B, Fabry's disease, CGD, ADAMTS13, Hurler's disease, X chromosome-mediated A-γ-globulinemia, adenosine deaminase-related immunodeficiency and respiratory distress syndrome in the newborn, which is linked with SP-B. Particularly preferably, the RNA, preferably the mRNA, according to the invention contains the coding sequence for surfactant protein B (SP-B) or for erythropoietin. Further examples of proteins which can be encoded by the RNA, preferably the mRNA, of the present invention according to the invention are growth factors such as human growth hormone hGH, BMP-2 or angiogenesis factors.

Alternatively, the RNA, preferably the mRNA, may contain a ribonucleotide sequence which encodes a full-length antibody or a smaller antibody (e.g., both heavy and light chains) which can be used in therapeutic settings to, e.g., confer immunity to a subject. Corresponding antibodies and their therapeutic application(s) are known in the art.

In another embodiment, the RNA, preferably the mRNA may encode a functional monoclonal or polyclonal antibody, which may be useful for targeting and/or inactivating a biological target (e.g., a stimulatory cytokine such as tumor necrosis factor). Similarly, the a RNA, preferably the mRNA sequence may encode, for example, functional anti-nephrotic factor antibodies useful for the treatment of membranoproliferative glomerulonephritis type II or acute hemolytic uremic syndrome, or alternatively may encode anti-vascular endothelial growth factor (VEGF) antibodies useful for the treatment of VEGF-mediated diseases, such as cancer.

Alternatively, the RNA, preferably the mRNA, may contain a ribonucleotide sequence which encodes an antigen which preferably can be used in therapeutic settings.

In another embodiment, the RNA, preferably the mRNA, may contain a ribonucleotide sequence which encodes a polypeptide or a protein which can be used in genome editing technologies. Genome editing is a type of genetic engineering in which DNA is inserted, deleted or replaced in the genome of an organism using nucleases. These nucleases create site-specific breaks at desired locations in the genome. The induced breaks are repaired by non-homologous end-joining or homologous recombination, resulting in targeted mutations in the genome, thereby "editing" the genome. The breaks may either be single-strand breaks or double-strand breaks (DSBs) while double-strand breaks (DSBs) are preferred. Numerous genome editing systems utilizing different polypeptides or proteins are known in the art, i.e., e.g., the CRISPR-Cas system, meganucleases, zinc finger nucleases (ZFNs) and transcription activator-like effector-based nucleases (TALEN). Methods for genome engineering are reviewed in Trends in Biotechnology, 2013, 31 (7), 397-405.

Thus, in a preferred embodiment, the RNA, preferably the mRNA, may contain a ribonucleotide sequence which encodes a polypeptide or protein of the Cas (CRISPR associated protein) protein family, preferably Cas9 (CRISPR associated protein 9). Proteins of the Cas protein family, preferably Cas9, may be used in CRISPR/Cas9 based methods and/or CRISPR/Cas9 genome editing technologies. CRISPR-Cas systems for genome editing, regulation and targeting are reviewed in Nat. Biotechnol., 2014, 32(4):347-355.

In another preferred embodiment, the RNA, preferably the mRNA, may contain a ribonucleotide sequence which encodes a meganuclease. Meganucleases are endodeoxyribonucleases which, in contrast to "conventional" endodeoxyribonucleases, recognize a large recognition site (e.g., a double-stranded DNA sequence of 12 to 40 base pairs). As a result, the respective site occurs only few times, preferably only once, in any given genome. Meganucleases are therefore considered to be the most specific naturally occurring restriction enzymes and, accordingly, are suitable tools in genome editing technologies.

In another preferred embodiment, the RNA, preferably the mRNA, contains a ribonucleotide sequence which encodes a zinc finger nuclease (ZFN). ZFNs are artificial restriction enzymes generated by fusing a zinc finger DNA-binding domain to a DNA-cleavage domain. Zinc finger domains can be engineered to target specific desired DNA sequences and this enables zinc-finger nucleases to target unique sequences within complex genomes. By taking advantage of the endogenous DNA repair machinery, ZFNs can be used to precisely alter the genome of higher organisms and are, therefore, suitable tools in genome editing technologies.

In another preferred embodiment, the RNA, preferably the mRNA, may contain a ribonucleotide sequence which encodes a transcription activator-like effector nuclease (TALEN). TALENs are restriction enzymes that can be engineered to cut specific sequences of DNA. TALENs are fusion proteins wherein a TAL effector DNA-binding domain is fused to a DNA cleavage domain of a nuclease. Transcription activator-like effectors (TALEs) can be engineered to bind practically any desired DNA sequence. Thus, when combined with a nuclease, DNA can be cut at specific desired locations.

Alternatively to the above, the RNA contains a ribonucleotide sequence which is not to be expressed as a protein or a polypeptide. Thus, the term RNA should not only be understood to mean any polynucleotide molecule which, if introduced into a cell, is translatable to a polypeptide/protein or fragment thereof. Rather, it is also contemplated that the RNA contains a ribonucleotide sequence which is only transcribed into a (functional) RNA, wherein said RNA is the final product (and, accordingly, does not require to be translated). In this context, it is envisaged that the RNA contains a ribonucleotide sequence which preferably provides the genetic information for an siRNA sequence or another desired ribonucleotide sequence.

It will be understood that the particles for use in the context of the present invention can comprise a single type of RNA, but may alternatively comprise a combination of two or more Types of RNA, e.g. in the form of particles comprising two or more types of RNA in single particles, or in the form of a blend of particles which differ in the type of RNA contained therein.

### Lipid Composition

The composition in accordance with the first aspect of the invention comprises particles which comprise RNA and a lipid composition.

The lipid composition comprises (i-a) a cholesterol derivative of formula (I) or a salt thereof, (i-b) a phosphoglyceride of formula (II) or a salt thereof, and (i-c) a pegylated phosphoglyceride of formula (III) or a salt thereof. Further lipid components may be present, but preferably components (i-a), (i-b) and (i-c) are the only lipid components in the lipid composition contained in the particles.

Component (i-a) of the lipid composition is a cholesterol derivative of formula (I) or a salt thereof: 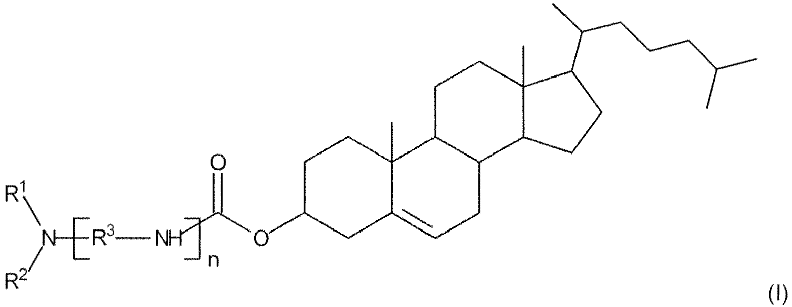 wherein
- n: is0or1,
- R¹: is a group -(CH₂)_{q}-NH₂ or a group -(CH₂)ᵣ-NH-(CH₂)ₛ-NH₂, wherein q, r and s are independently an integer of 2 to 6,
- R²: is a group -(CH₂)ₜ-NH₂ or a group -(CH₂)ᵤ-NH-(CH₂)_{w}-NH₂, wherein t, u and w are independently an integer of 2 to 6,
- R³: is a linear alkanediyl group having 1 to 4 carbon atoms.

Compounds of this formula and their preparation are described e.g. in US 5,783,565.

The integer n in formula (I) is preferably 0.

The integers q, r, s, t, u and w are preferably independently selected from 3 and 4.

It is also preferred that R¹ is a group -(CH₂)_{q}-NH₂ and R² is a group a group -(CH₂)ₜ-NH₂ or a group -(CH₂)ᵤ-NH-(CH₂)_{w}-NH₂, or that R² is a group a group -(CH₂)ₜ-NH₂, and R¹ is a group -(CH₂)_{q}-NH₂ or a group -(CH₂)ᵣ-NH-(CH₂)ₛ-NH₂. In both cases, n is also preferably 0.

Salt forms of the cholesterol derivative of formula (I) may be provided by protonating one or more of the amino groups contained in the compound with an acid so that the compound carries a cationic charge. As will be appreciated, a protonated amino group is one wherein an additional hydrogen atom is bound to the nitrogen atom of the amino group, such that a tretravalent nitrogen carrying a cationic charge results. Suitable nitrogen atoms in the compound of formula (I) are the nitrogen atom contained in the group -N(R¹)(R²), and the nitrogen atoms contained in R¹ and in R², respectively. It will be understood that the anion acting as a counterion for any protonated amino group is generally a physiologically acceptable anion. In the compositions of the present invention, the compound of formula (I) will typically form a salt with the acidic groups of the RNA. However, other counterions are not excluded, and examples include chloride, bromide, iodide, sulfate, nitrate, phosphate, hydrogenphosphate, dihydrogenphosphate, carbonate, and hydrogencarbonate anions.

As will be understood by the skilled reader, the compound of formula (I) as a cholesterol derivative can be illustrated by the preferred formula (Ia): 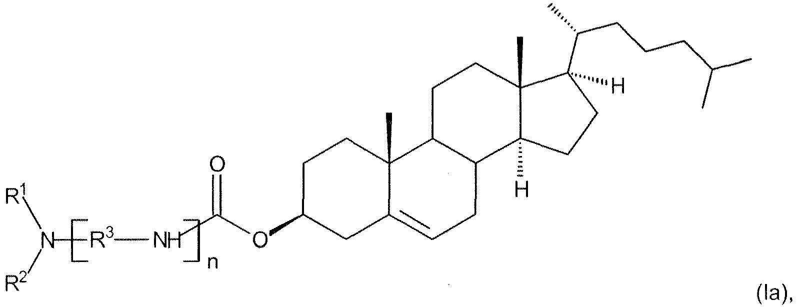 or the still more preferred formula (Ib):

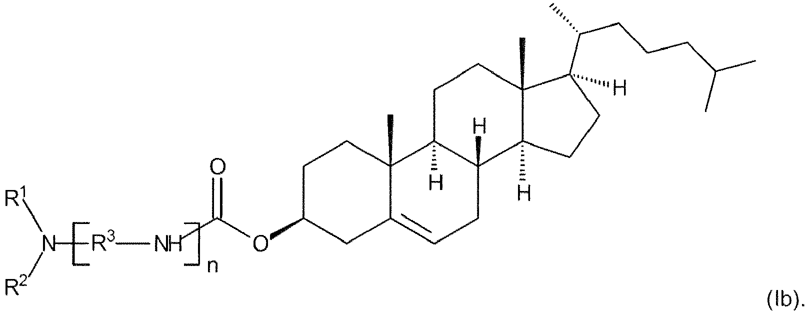

In formulae (Ia) and (Ib), the definitions and preferred definitions for R¹, R², R³ and n given above with respect to formula (I) continue to apply.

Most preferred as component (i-a) of the lipid composition is GL67 or a salt thereof, wherein, with respect to formula (I), (Ia) and (Ib), n is 0, R¹ is a group -(CH₂)₃-NH₂ and R² is a group -(CH₂)₄-NH-(CH₂)₃-NH₂.

Component (i-b) of the lipid composition is a phosphoglyceride of formula (II) or a salt thereof: 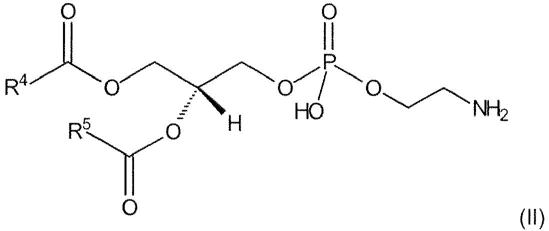 wherein
- R⁴: is a linear alkyl group having 10 to 24 carbon atoms or a linear alkenyl group having 1 to 3 double bonds and 10 to 24 carbon atoms;
- R⁵: is a linear alkyl group having 10 to 24 carbon atoms or a linear alkenyl group having 1 to 3 double bonds and 10 to 24 carbon atoms.

Preferably, R⁴ and R⁵ are each a linear alkyl group having 14 to 20 carbon atoms or are each a linear alkenyl group having one or two double bonds and 14 to 20 carbon atoms. More preferably R⁴ and R⁵ are each a linear alkenyl group having one double bond and 14 to 20 carbon atoms.

Salt forms of the compound of formula (II) include internal salt forms wherein the proton of the acidic -OH group attached to the P atom shown in formula (II) protonates the amino group shown therein. Such salt forms also include salts formed by the acidic -OH group with a base, or salts formed by the amino group with an acid. Again, it will be understood that the salts are generally salts which are pharmaceutically acceptable. As salts formed with a base, mention may be made of alkali metal salts such as sodium or potassium salts; alkaline-earth metal salts such as calcium or magnesium salts and ammonium salts. As exemplary salts formed with an acid, mention may be made of a salt formed with the acidic groups of the RNA, but other salts are not excluded, and mineral acid salts such as chloride, bromide, or iodide, sulfate salts, nitrate salts, phosphate salts, hydrogenphosphate salts, or dihydrogenphosphate salts, carbonate salts, and hydrogencarbonate salts may be mentioned as examples.

Most preferred as component (i-b) of the lipid composition is 1,2-dioleoyl-*sn*-glycero-3-phosphoethanolamine (DOPE), or a salt thereof.

Component (i-c) of the lipid composition is a pegylated phosphoglyceride of formula (III) or a salt thereof: 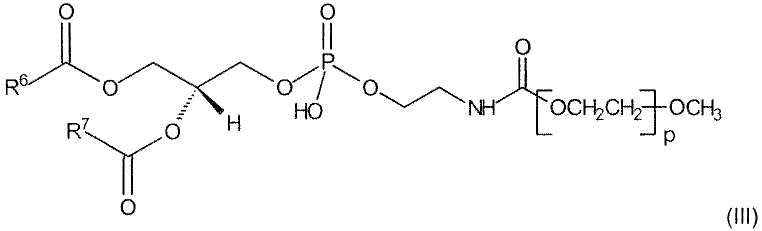 wherein
- p: is an integer of 5 to 200, preferably 10 to 170 and most preferably 10 to 140;
- R⁶: is a linear alkyl group having 10 to 20 carbon atoms or a linear alkenyl group having 1 to 3 double bonds and 10 to 20 carbon atoms;
- R⁷: is a linear alkyl group having 10 to 20 carbon atoms or a linear alkenyl group having 1 to 3 double bonds and 10 to 20 carbon atoms.

Preferably, R⁶ and R⁷ are each a linear alkyl group having 10 to 20 carbon atoms, more preferably, R⁶ and R⁷ are each a linear alkyl group having 10 to 16 carbon atoms.

Thus, it is also preferred that, in formula (III), R⁶ and R⁷ are each a linear alkyl group having 10 to 16 carbon atoms and p is an integer of 10 to 140.

Salt forms of the compound of formula (III) are generally salts which are pharmaceutically acceptable. Typical salts of the compound of formula (III) are salts formed with a base, and mention may be made e.g. of alkali metal salts such as sodium or potassium salts; alkaline-earth metal salts such as calcium or magnesium salts and ammonium salts.

Strongly preferred as a component (i-c) in the lipid composition is 1,2-dimyristoyl-*sn*-glycero-3-phosphoethanolamine-PEG (DMPE-PEG) or a salt thereof wherein the PEG (polyethylene glycol) moiety contains 10 to 140 repeating units, i.e. with respect to formula (III), R⁶ and R⁷ are each a linear alkyl group having 13 carbon atoms, and p is an integer of 10 to 140 in this strongly preferred embodiment. Most preferred as a component (i-c) is 1,2-dimyristoyl-*sn-*glycero-3-phosphoethanolamine-PEG5000 (DMPE-PEG5000) or a salt thereof, wherein 5000 indicates the number average molecular weight of the PEG moiety, corresponding to an average value of p of about 113.

Thus, it will be appreciated that a particularly preferred lipid composition for use in the context of the present invention is one wherein:
component (i-a) is GL67 or a salt thereof,
component (i-b) is 1,2-dioleoyl-*sn*-glycero-3-phosphoethanolamine (DOPE) or a salt thereof, and
component (i-c) is 1,2-dimyristoyl-*sn*-glycero-3-phosphoethanolamine-PEG (DMPE-PEG) or a salt thereof, wherein the PEG (polyethylene glycol) moiety contains 10 to 140 repeating units, and is more preferably 1,2-dimyristoyl-*sn*-glycero-3-phosphoethanolamine-PEG5000 (DMPE-PEG5000) or a salt thereof.

In the lipid composition contained in the particles of the present composition, the molar ratio of the components (i-a), (i-b) and (i-c), i.e. (i-a) : (i-b) : (i-c), is preferably 1 : (0.5 to 5) : (0.01 to 1), more preferably 1 : (1 to 5) : (0.01 to 0.5) and most preferably 1 : (1 to 3) : (0.02 to 0.2).

The lipid composition preferably contains the components (i-a), (i-b) and (i-c) as the only lipid components.

In line with the above, a strongly preferred composition in accordance with the first aspect of the invention is one which comprises:
(i) particles contained in a liquid phase, wherein the particles comprise mRNA and a lipid composition, and wherein the lipid composition comprises:
   (i-a) GL67 or a salt thereof,
   (i-b) 1,2-dioleoyl-*sn*-glycero-3-phosphoethanolamine (DOPE) or a salt thereof, and
   (i-c) 1,2-dimyristoyl-*sn*-glycero-3-phosphoethanolamine-PEG (DMPE-PEG) or a salt thereof, wherein the PEG (polyethylene glycol) moiety contains 10 to 140 repeating units, and is more preferably 1,2-dimyristoyl-*sn*-glycero-3-phosphoethanolamine-PEG5000 (DMPE-PEG5000) or a salt thereof,
   in a molar ratio of the components (i-a) : (i-b) : (i-c) of 1 : (1 to 3) : (0.02 to 0.2); and
(ii) 1,2-propanediol.

### Particles

The composition in accordance with the first aspect of the invention comprises particles comprising RNA and the lipid composition as discussed above.

The particles typically comprise nanoparticles comprising RNA and the lipid composition or microparticles comprising RNA and the lipid composition. As will be understood by the skilled reader, the "or" is used in this context in a non-exclusive manner, unless specifically indicated otherwise. Thus, the reference to nano- or microparticles encompasses compositions containing nanoparticles, compositions containing microparticles, and compositions containing both nanoparticles and microparticles. As used herein, the term nanoparticles refers generally to particles with a diameter in the nanometer size range, i.e. a diameter of 1 nm or more and below 1000 nm. The term microparticles refers generally to particles with a diameter in the micrometer size range, i.e. a diameter of 1000 nm or more and 100 µm or less. Preferably, the particles consist of nanoparticles comprising RNA and the lipid composition or microparticles comprising RNA and the lipid composition.

The particles contained in the compositions in accordance with the present invention preferably show an average particle diameter in the range of 1 to 5000 nm, more preferably 10 to 4000 nm, and most preferably 50 to 3000 nm.

The upper limit for the diameter of the single particles in the compositions in accordance with the invention is preferably 20 µm, more preferably 10 µm and most preferably 5 µm. Thus, as will be understood from the above, a strongly preferred particle formulation would be one with an average particle diameter in the range of 50 to 3000 nm, and particles with a maximum particle diameter of 5 µm.

The particle diameters and the average particle diameter of the nano- or microparticle formulation as referred to herein can be conveniently determined via dynamic light scattering (DLS). Generally, the diameters and the average diameter as referred to herein are indicated as hydrodynamic diameters of the particles in a suspended state determined via dynamic light scattering. Since the effect of temperature is taken into account by the measurement equipment (e.g. Malvern ZetaSizer) when reporting the results, the measured diameters are generally not temperature dependent. However, the measurement is typically carried out at room temperature (25 °C). As a suspension medium for DLS measurements, for example water or water containing 1,2-propanediol can be used, as appropriate.

In cases where an average particle size or an average particle diameter is indicated, the average is typically the z-average unless indicated otherwise.

In the particles contained in the compositions in accordance with the invention, acidic groups of the RNA will typically protonate amino groups contained in the lipid formulation, so that anionic RNA molecules and cationic lipid molecules can interact, preferably resulting in the formation of complexes between the RNA molecules and the lipid molecules.

The N/P ratio of the number of nitrogen atoms N derived from the cholesterol derivative of formula (I) in the lipid composition to the number of phosphate groups P in the RNA is preferably in the range of 1 to 100, more preferably 1 to 30, and most preferably 2 to 20.

Preferably, the particles have the form of liposomes or of lipid nanoparticles.

In line with the above, a strongly preferred composition in accordance with the first aspect of the invention is one which comprises:
(i) nano- or microparticles contained in a liquid phase, wherein the nano- or microparticles comprise mRNA and a lipid composition, wherein the lipid composition comprises:
   (i-a) GL67 or a salt thereof,
   (i-b) 1,2-dioleoyl-*sn*-glycero-3-phosphoethanolamine (DOPE) or a salt thereof, and
   (i-c) 1,2-dimyristoyl-*sn*-glycero-3-phosphoethanolamine-PEG (DMPE-PEG) or a salt thereof, wherein the PEG (polyethylene glycol) moiety contains 10 to 140 repeating units, and is more preferably 1,2-dimyristoyl-*sn*-glycero-3-phosphoethanolamine-PEG5000 (DMPE-PEG5000) or a salt thereof,
   in a molar ratio of the components (i-a) : (i-b) : (i-c) of 1 : (1 to 3) : (0.02 to 0.2), and wherein the N/P ratio of the number of nitrogen atoms N derived from the cholesterol derivative of formula (I) in the lipid composition to the number of phosphate groups P in the mRNA is 2 to 20; and
(ii) 1,2-propanediol.

In addition to the RNA and the lipid composition, the particles in the compositions in accordance with the present invention may comprise one or more further components, e.g. excipients or additives which are typically pharmaceutically acceptable components. For example, such further components may facilitate the transport to specific sites or promote the further uptake of the particles into specific sites after they have been administered to a patient, or they may help to stabilize the particles or the therapeutically active agent contained therein.

Besides the RNA and the lipid composition, the particles contained in the compositions in accordance with the first aspect of the invention may comprise, as an optional additive or as optional additives, one or more components that exert an effector function during delivery of the therapeutic agent, and preferably during the delivery of a ribonucleic acid as a therapeutic agent to and into a cell. Such components can be, but are not limited to, polyanions, further lipids, shielding oligomers or polymers, poloxamers (also known as pluronics), poloxamines, targeting ligands, endosomolytic agents, cell penetrating and signal peptides, magnetic and non-magnetic nanoparticles, RNAse inhibitors, fluorescent dyes, radioisotopes or contrast agents for medical imaging. The term "effector function" encompasses any function that supports achieving an intended biological effect of the therapeutically active agent of the composition at or in a biological target or the surroundings of a biological target. For example, compositions for nucleic acid delivery have been formulated to comprise non-coding nucleic acids or non-nucleic acid polyanions as stuffer materials (Kichler et al. 2005, J Gene Med, 7, 1459-1467). Such stuffer materials are suitable for reducing the dose of a nucleic acid having an intended biological effect while maintaining the extent or degree of that effect obtained at a higher nucleic acid dose in the absence of such stuffer material. Non-nucleic acid polyanions have also been used to obtain prolonged in vivo gene expression at reduced toxicity (Uchida et al. 2011, J Control Release, 155, 296-302).

Other exemplary shielding polymers described in the literature which may be useful components for a particle formulation comprising a complex of a ribonucleic acid with a cationic excipient include hydroxyethyl starch (HES; Noga et al. Journal of Controlled Release, 2012. 159(1): 92-103, a PAS-/PA-polypeptide (Pro, Ala, Ser (or Pro, Ala) polypeptide: Schlapschy et a. Protein Eng Des Sel. 2013 Aug;26(8):489-501 or Polysarcosine (Psar,: Heller et al. Macromol Biosci 2014; 14: 1380-1395).

Targeting ligands may be useful e.g. in particle formulations for ribonucleic acid delivery for preferential and improved transfection of target cells (Philipp and Wagner in "Gene and Cell Therapy - Therapeutic Mechanisms and Strategy", 3rd Edition, Chapter 15. CRC Press, Taylor & Francis Group LLC, Boca Raton 2009). A targeting ligand can be any compound that confers to compositions of the present invention a target recognition and/or target binding function in a direct or indirect manner. Exemplary targeting ligands are the prostacycline analoga disclosed in WO 2011/076391, such as Iloprost or Treprostinil. An antibody may also act as a targeting ligand. As ligands for particles, folic acid and N-acetyl galactosamine can be mentioned. In most general terms, a target is a distinct biological structure to which a targeting ligand can bind specifically via molecular interaction and where such binding will ultimately lead to preferential accumulation of the therapeutic agent, such as a nucleic acid, comprised in the composition in a target tissue and/or at or in a target cell. Targeting ligands can be coupled, e.g. to the terminal ends of the shielding polymer.

Furthermore, endosomolytic agents such as endosomolytic peptides (Plank et al. 1998, Adv Drug Deliv Rev, 34, 21-35) or any other compound that is suited to enhance the endosomal release of an endocytosed nucleic acid are useful components of compositions of present inventions. Similarly, cell penetrating peptides (in another context also known as protein transduction domains) (Lindgren et al. 2000, Trends Pharmacol Sci, 21, 99-103) can be useful components of the composition of the present invention in order to mediate intracellular delivery of a nucleic acid. The so-called TAT peptide falls within this class and also has nuclear localization function (Rudolph et al. 2003, J Biol Chem, 278, 11411-11418).

Preferably, the particles have an active load, expressed as the weight of the RNA as therapeutically active agent to the total weight of the particles in the particle formulation, in the range of 0.1 to 95 %(w/w), more preferably 0.5 to 80 %(w/w), most preferably 1 to 50 %(w/w).

### Composition comprising the particles and 1,2-propanediol

The composition in accordance with the first aspect of the invention comprises particles comprising RNA and the lipid composition together with 1,2-propanediol. As will be appreciated, the information above regarding suitable and preferred embodiments of the RNA, of the lipid composition and of the particles comprising them continues to apply in this context of the following discussion.

Since the composition in accordance with the first aspect of the invention contains RNA as a therapeutically active agent and is suitable for the administration of the therapeutically active agent to a patient, it can be referred to as therapeutic composition or pharmaceutical composition.

In the composition according to the first aspect of the present invention which comprises the particles comprising RNA and the lipid composition in a liquid phase, the particles comprising RNA and the lipid composition are preferably dispersed in the liquid phase. As implied by the term "dispersed", the particles form a discontinuous phase in the continuous liquid phase in this case. Generally, it is preferred that the dispersion is provided as a two-phase dispersion with one continuous liquid phase and the particles comprising RNA and the lipid composition dispersed as a discontinuous phase therein.

The composition in accordance with the first aspect of the invention, wherein the particles comprising RNA and the lipid composition are contained in a liquid phase, preferably comprises the particles in an amount so as to provide the RNA contained in the particles at a concentration of 0.01 to 50 mg/ml, more preferably 0.02 to 30 mg/ml, and most preferably 0.05 to 10 mg/ml, based on the total volume of the composition.

Typically, the 1,2-propanediol is contained as a further component in the liquid phase of the composition in accordance with the first aspect of the invention, wherein the particles comprising RNA and the lipid composition are contained, preferably suspended. The 1,2-propanediol is preferably dissolved in the liquid phase. However, it may also be partly associated with the particles contained in the liquid phase.

In the composition in accordance with the first aspect of the present invention comprising the particles contained in a liquid phase, the 1,2-propanediol is preferably present at a concentration of 0.1 to 50 % w/v, more preferably 0.2 to 30 % w/v, most preferably 0.3 to 20 % w/v, where the percentage value indicates the weight of the 1,2-propanediol in g per 100 ml of the total volume of the composition.

The liquid phase wherein the particles are contained, preferably dispersed, typically contains water as a solvent. Preferably, 50 % or more, more preferably 70 % or more by volume (based on the total volume of the liquid phase at 20°C) are provided by water. More preferably, water and the 1,2-propanediol are the only solvents contained in the liquid phase.

As exemplary further optional additives of the liquid phase, one or more selected from salts, sugars, organic solvents and buffers may be mentioned. Buffers can be used which are conventionally used in pharmaceutical or biological applications.

In line with the above, a strongly preferred composition in accordance with the first aspect of the invention is one which comprises:
(i) nano- or microparticles dispersed in a liquid phase comprising water as a solvent, wherein the nano- or microparticles comprise mRNA and a lipid composition, wherein the lipid composition comprises:
   (i-a) GL67 or a salt thereof,
   (i-b) 1,2-dioleoyl-*sn*-glycero-3-phosphoethanolamine (DOPE) or a salt thereof, and
   (i-c) 1,2-dimyristoyl-*sn*-glycero-3-phosphoethanolamine-PEG (DMPE-PEG) or a salt thereof, wherein the PEG (polyethylene glycol) moiety contains 10 to 140 repeating units, and is more preferably 1,2-dimyristoyl-*sn*-glycero-3-phosphoethanolamine-PEG5000 (DMPE-PEG5000) or a salt thereof,
   in a molar ratio of the components (i-a) : (i-b) : (i-c) of 1 : (1 to 3) : (0.02 to 0.2), and wherein the N/P ratio of the number of nitrogen atoms N derived from the cholesterol derivative of formula (I) in the lipid composition to the number of phosphate groups P in the mRNA is 2 to 20; and
(ii) 1,2-propanediol as a further component in the liquid phase wherein the nano-or microparticles are suspended; and wherein the composition contains the 1,2-propanediol in an amount of 0.3 to 20 % w/v of the 1,2-propanediol, based on the total volume of the composition.

### Pharmaceutical Applications

As already mentioned above, the RNA molecules, preferably the mRNA molecules, which are present in the compositions in accordance with the present invention as defined above are particularly useful in a medical setting and in the treatment of certain diseases, and, in particular, in RNA-based therapies. Thus, the compositions in accordance with the invention comprising the RNA molecules are suitable as pharmaceutical compositions.

In particular the composition in accordance with the first aspect of the present invention is suitable for administration to a subject. Thus, the RNA, preferably the mRNA, contained therein can also be delivered to the subject. A preferred route of administration for the composition is the administration to or via the respiratory tract, in particular pulmonary administration or nasal administration.

However, it will be appreciated by the skilled reader that the composition in accordance with the first aspect of the invention can also be administered via other routes of administration which are known in the art for particle formulations of a therapeutically active agent, such as the intravenous administration in the form of a dispersion.

Devices for forming an aerosol from a composition comprising particles contained in a liquid or for nebulising such a composition are known in the art and are commercially available. They can be used in order to accomplish the administration of the composition in accordance with the first aspect of the invention to or via the respiratory tract, in particular pulmonary administration. For the administration via the nose, for example a nasal spraying device or nasal infusion may be used.

Thus, a further aspect of the present invention relates to a device for forming an aerosol from a particulate composition contained in a liquid or for nebulising such a composition, which device comprises the composition in accordance with the first aspect of the present invention. The device is preferably an inhaler selected from a metered dose inhaler, a nebulizer, and a nasal spraying device.

Via such an administration to a subject, the RNA contained in the particles may be delivered to target cells in or via the respiratory tract. The term "delivered to target cells" preferably means transfer of the RNA, preferably single-stranded RNA such as mRNA, into the cell.

The composition can be administered to the subject at a suitable dose. The dosage regimen will be determined by the attending physician and clinical factors. As is well known in the medical arts, dosages for any one subject depend upon many factors, including the subject's size, body surface area, age, the particular compound to be administered, sex, time and route of administration, general health, and other drugs being administered concurrently. A typical dose of therapeutically active substances can be, for example, in the range of 1 ng to several grams. The dosage of an (m)RNA for expression or for inhibition of expression should correspond to this range; however, doses below or above this exemplary range are envisioned, especially considering the aforementioned factors. Generally, the regimen as a regular administration of the pharmaceutical composition should be in the range of 0.01 µg to 10 mg units per kilogram of body weight per day. If the regimen is a continuous infusion, it should also be in the range of 1 µg to 10 mg units per kilogram of body weight, respectively. Progress can be monitored by periodic assessment. Dosages will vary but a preferred dosage for administration of (m)RNAs as constituents of the composition of the present invention is from approximately 10⁶ to 10¹⁹ copies of the (m)RNA molecule.

Also made available by the present invention is a method of treatment, comprising administering the composition in accordance with the first aspect of the invention to a patient, preferably via administration to or via the respiratory tract, more preferably via pulmonary administration or nasal administration. Thus, the RNA contained in said composition can cause a preventive or therapeutic effect. Notably, the term "patient" comprises animals and humans.

By administering the composition of the present invention, in particular the first aspect of the invention, to a subject, diseases can be treated or prevented. The term "disease" refers to any conceivable pathological condition that can be treated, prevented or vaccined against by employing the composition of the present invention. Said diseases may e.g. be inherited, acquired, infectious or non-infectious, age-related, cardiovascular, metabolic, intestinal, neoplastic (in particular cancer) or genetic. A disease can be based, for example, on irregularities of physiological processes, molecular processes, biochemical reactions within an organism that in turn can be based, for instance, on the genetic equipment of an organism, on behavioural, social or environmental factors such as the exposure to chemicals or radiation.

The terms "treatment" or "treating" used herein generally mean obtaining a desired pharmacological and/or physiological effect. Accordingly, the treatment of the present invention may relate to the treatment of (acute) states of a certain disease but may also relate to the prophylactic treatment in terms of completely or partially preventing a disease or symptom thereof. Preferably, the term "treatment" is to be understood as being therapeutic in terms of partially or completely curing a disease and/or adverse effects and/or symptoms attributed to the disease. "Acute" in this respect means that the subject shows symptoms of the disease. In other words, the subject to be treated is in actual need of a treatment and the term "acute treatment" in the context of the present invention relates to the measures taken to actually treat the disease after the onset of the disease or the breakout of the disease. The treatment may also be prophylactic or preventive treatment, i.e., measures taken for disease prevention, e.g., in order to prevent the infection and/or the onset of the disease. Therapeutic progress can be monitored by periodic assessment.

Generally, the RNA, preferably the mRNA, is included in an effective amount in the composition in accordance with the present invention. The term "effective amount" refers to an amount sufficient to induce a detectable therapeutic response in the subject to which the pharmaceutical composition is to be administered. In accordance with the above, the content of the RNA, preferably the mRNA, in accordance with the above first aspect of the present invention in the pharmaceutical composition is not limited as far as it is useful for treatment as described above. As noted above, the composition in accordance with the first aspect of the invention, wherein the particles comprising RNA and the lipid composition are contained in a liquid phase, preferably comprises the particles in an amount so as to provide the RNA contained in the particles at a concentration of 0.01 to 50 mg/ml, more preferably 0.02 to 30 mg/ml, and most preferably 0.05 to 10 mg/ml, based on the total volume of the composition.

In accordance with this invention, the term "pharmaceutical composition" relates to a composition in accordance with the invention, in particular a composition in accordance with the first aspect of the present invention, for administration to a subject. Exemplary subjects include a mammal such as a dog, cat, pig, cow, sheep, horse, rodent, e.g., rat, mouse, and guinea pig, or a primate, e.g., gorilla, chimpanzee, and human. In a most preferable embodiment, the subject is a human.

The pharmaceutical composition of the present invention may be for use in RNA-based therapies. As mentioned above, the RNA molecule, preferably the mRNA molecule, comprises a sequence encoding a protein and, accordingly, can be used in RNA-based therapies wherein the RNA, preferably the mRNA, encodes a therapeutically or pharmaceutically active polypeptide or protein having a therapeutic or preventive effect. Thus, in preferred embodiments, the pharmaceutical composition of the present invention may be for use in RNA-based therapies in the treatment or prevention of a disease as recited in the above **Table 2.** Accordingly, RNA-based therapies in accordance with the present invention may be for use in the treatment or prevention of a disease as recited in the above **Table 2.**

Thus, the pharmaceutical composition of the present invention may be for use in RNA-based therapies in cases where the gene defects described in the above **Table 2** lead to a disease which can then be treated or prevented by a transcript replacement therapy/enzyme replacement therapy with the RNA molecule, preferably the mRNA molecule, of the present invention, wherein the RNA molecule encodes an intact version of the protein or a functional fragment thereof compensating the disclosed defective gene.

In other embodiments, the pharmaceutical composition of the present invention may be for use in RNA-based therapies in accordance with the present invention wherein the RNA, preferably the mRNA, encodes a therapeutically or pharmaceutically active polypeptide, protein or peptide having a therapeutic or preventive effect, wherein said polypeptide, protein or peptide is selected from the group encoded by the genes as outlined in **Table 2.**

The pharmaceutical composition of the present invention is particularly suitable for use in RNA-based therapies in the treatment or prevention of lung diseases. As exemplary diseases, Alpha-1-antitrypsin, Asthma, Cystic fibrosis, Surfactant metabolism dysfunction or Primary ciliary dyskinesia as recited in the above Table 2 may be mentioned.

In other exemplary embodiments, the pharmaceutical composition of the present invention may be for use in RNA-based therapies in the treatment or prevention of lysosomal diseases like Gaucher disease, Fabry disease, MPS I, MPS II (Hunter syndrome), MPS VI and Glycogen storage diseases such as for example Glycogen storage disease type I (von Gierecke's disease), type II (Pompe's disease), type III (Cori's disease, type IV (Andersen's disease, type V (McArdle's disease, type VI (Hers disease), type VII (Tauri's disease), type VII, type IX, type X, type XI (Fanconi-Bickel syndrome), type XI, or type 0. Transcript replacement therapies/enzyme replacement therapies beneficially do not affect the underlying genetic defect, but increase the concentration of the enzyme in which the patient is deficient. As an example, in Pompe's disease, the transcript replacement therapy/enzyme replacement therapy replaces the deficient Lysosomal enzyme acid alpha-glucosidase (GAA).

In accordance with further examples, RNA-based therapies in accordance with the present invention may be for use in treating cancer, a cardiovascular disease, a viral infection, an immune dysfunction, an autoimmune disease, a neurologic disorder, an inherited metabolic disorders or a genetic disorder or any disease where a protein or protein fragment produced in a cell may have a beneficial effect for the patent. Examples of cancer include head and neck cancer, breast cancer, renal cancer, bladder cancer, lung cancer, prostate cancer, bone cancer, brain cancer, cervical cancer, anal cancer, colon cancer, colorectal cancer, appendix cancer, eye cancer, gastric cancer, leukemia, lymphoma, liver cancer, skin cancer, ovarian cancer, penile cancer, pancreatic cancer, testicular cancer, thyroid cancer, vaginal cancer, vulvar cancer, endometrial cancer, cardiac cancer and sarcoma. Examples of cardiovascular diseases include atherosclerosis, coronary heart disease, pulmonary heart disease and cardiomyopathy. Examples of immune dysfunctions and autoimmune diseases include, but are not limited to, rheumatic diseases, multiple sclerosis and asthma. Examples of viral infections include, but are not limited to, infections with human immunodeficiency virus, herpes simplex virus, human papillomavirus as well as hepatitis B and C virus. Examples of neurologic disorders include, but are not limited to, Parkinson's disease, multiple sclerosis, and dementia. Examples of inherited metabolic disorders include, but are not limited to, Gaucher's disease and Phenylketonuria.

### Processes for Preparation

Suitable techniques for the provision of particles comprising a nucleic acid and a lipid composition in a liquid phase are available to the skilled person, and can be adapted in order to provide the compositions in accordance with the first aspect of the invention wherein the particles comprise RNA together with the lipid composition discussed above.

For example, liposomes as particles comprising RNA and the lipid composition can be conveniently provided via rehydration of the lipid composition, such as the rehydration of lipid films, followed by homogenization techniques like e.g. ultra sonication or extrusion, where required. An alternative approach is the infusion of a lipid composition dissolved in an organic solvent into water or an aqueous solution. As an exemplary method, which can be relied on e.g. for the formation of lipid nanoparticles, the solvent displacement method can be mentioned.

As an alternative method for the provision of compositions in accordance with the invention wherein particles are contained in a liquid phase, precipitation methods, such as nanoprecipitation of the particles, may be mentioned.

The 1,2-propanediol may be conveniently added to a liquid phase wherein particles comprising RNA and a lipid composition are contained, or wherein the particles are to be provided. The addition of the 1,2-propanediol to the liquid phase may be accomplished at one or more of various stages including prior to, during or after providing the particles comprising RNA and the lipid composition in the liquid phase.

Thus, as noted above, a further aspect of the invention relates to a process for the preparation of a composition in accordance with the first aspect of the invention, said process comprising the steps of:
a) dissolving and mixing the components of the lipid composition in an organic solvent, followed by the lyophilization of the lipid composition;
b) rehydrating the lyophilized lipid composition via addition of water;
c) combining the rehydrated lipid composition with an aqueous solution of the RNA to allow particles comprising RNA and the lipid composition to be formed which are contained in a liquid phase; and
d) adding 1,2-propanediol.

The addition of the 1,2-propanediol may be conveniently accomplished e.g. by adding it together with water in step b), by adding it to the rehydrated lipid composition following step b), by adding it together with the aqueous solution of the RNA in step c), or by adding it to the liquid phase following step c), wherein the particles comprising RNA and the lipid composition are contained. It will be appreciated that the addition can be carried out in one step or in multiple steps, e.g. at different stages of the process.

A solid composition (not claimed in the context of the present invention) can be prepared by a process, comprising:
a first step of preparing a composition in accordance with the above first aspect by a process comprising
   a) dissolving and mixing the components of the lipid composition in an organic solvent, followed by the lyophilization of the lipid composition;
   b) rehydrating the lyophilized lipid composition via addition of water;
   c) combining the rehydrated lipid composition with an aqueous solution of the RNA to allow particles comprising RNA and the lipid composition to be formed which are contained in a liquid phase; and
   d) adding 1,2-propanediol;
and a second step of freezing the composition obtained in the first step.

Also in this process, the addition of the 1,2-propanediol may be conveniently accomplished e.g. by adding it together with water in step b), by adding it to the rehydrated lipid composition following step b), by adding it together with the aqueous solution of the RNA in step c), or by adding it to the liquid phase following step c), wherein the particles comprising RNA and the lipid composition are contained. It will be appreciated that the addition can be carried out in one step or in multiple steps, e.g. at different stages of the process.

The freezing step as a second step is typically accomplished by subjecting the suspension composition to sufficiently cold temperatures (e.g. -10°C or less, preferably -20°C or less) in a suitable container. As a cooling medium, e.g. cold air or cold liquids may be used.

Incidentally, as explained above, the solid composition is a composition which allows particles comprising RNA to be stored. To that extent, it will be understood that a composition in accordance with the first aspect of the invention can be recovered from the solid composition by thawing the solid composition.

Thus, as a further aspect, the present invention also provides a process for the preparation of a composition in accordance with the above first aspect, said process comprising:
a first step of preparing a composition in accordance with the above first aspect by a process comprising
   a) dissolving and mixing the components of the lipid composition in an organic solvent, followed by the lyophilization of the lipid composition;
   b) rehydrating the lyophilized lipid composition via addition of water;
   c) combining the rehydrated lipid composition with an aqueous solution of the RNA to allow particles comprising RNA and the lipid composition to be formed which are contained in a liquid phase; and
   d) adding 1,2-propanediol;
a second step of freezing the composition obtained in the first step,
and a third step of thawing the frozen composition obtained in the second step.

### Examples

### Abbreviations

| Abbreviation | Description |
|---|---|
| RT | Room temperature |
| mRNA | Messenger ribonucleic acid |
| FLuc | Firefly luciferase |
| w/o | without |
| cmRNA | chemically modified ribonucleic acid |
| FLuc | Firefly luciferase |
| PG | 1,2-propanediol, propylene glycol |
| N/P | Number of nitrogen atoms in cholesterol derivative (I) to number of phosphate groups in mRNA |

### Example I: Application of complexes to ALI cultures

### Methods:

Formulation of RNA-Lipid complex at N/P5 (and N/P3):
Stock solutions of GL67, DOPE and DMPE-PEG5000 were produced at a concentration of 10 - 20 mg/mL each in at 9:1 mix of t-butanol and water. Lipids were mixed in a molar ratio of 1:2:0.05 (GL67:DOPE:DMPE-PEG5000) at a total lipid mass of 5mg, frozen at -80°C over night and lyophilized for at least 96h. Before usage the dry lipid mix was rehydrated by addition of 400µL 5% (v/v) propylene glycol (resulting in a total lipid concentration of 12.5mg/mL) followed by incubation at room temperature for 30min without shaking, vortexing for 10s followed by ultrasonication in a water bath at 37°C for 10min. The generated liposome-mix was diluted for complexation with mRNA. For this purpose 69.65µL (87.69µL for N/P3) water was mixed with 10.24µL propylene glycol and vortexed for 3s. 45.10µL (27.06µL for N/P3) liposome-mix was added and the mixture was vortexed for 10 s and equilibrated at 39°C for 3min. 125µL mRNA solution in water (c: 0.5mg/mL) was equilibrated at 39°C for 3 min. For complex assembling the mRNA dilution was soaked into the fine needle syringe (BD Micro-Fine Insuline syringe 0.5 mL U40 8 mm, 07468060 Becton Dickinson), quickly injected into the liposome dilution, immediately vortexed for 10s and incubated for 10min at RT for complex assembling. The complexes were then stored on ice.

### Treatment of ALI cultures

MucilAir^{™}-ALI were obtained from Epithelix (France). 5 days prior the transfection a mucus wash was performed, to remove the accumulated mucus on the apical surface. 200µL of PBS^{-/-} were added on the apical side and incubated for 20min at 37°C. To detach the mucus from the apical surface 3 back and forth movements were done with 100µL from the apical liquid with a pipet. PBS^{-/-} from the apical surface of MucilAir^{™} was removed by gentle aspiration without damaging the epithelium. At the day of transfection the ALI cultures were again washed with 200µL PBS^{-/-} followed by a washing step with 200µL of the corresponding diluent of the mRNA complex (water or 5% propylene glycol). The diluent was removed immediately from the apical surface of the ALI cultures by gentle aspiration without damaging the epithelium. Then, each well was transfected with 3µg total complexed mRNA in a volume of 50µL. The formulations were diluted with the corresponding diluent, added to the apical surface of the ALI-culture and incubated for 6h at 37°C and 5% CO₂. Afterwards the complex solution was removed and the cell layer washed with PBS^{-/-} and maintained as ALI culture for 24h at 37°C and 5% CO₂.

### Detection of tdTomato via fluorescence microscopy:

Fluorescence imaging was used to detect positive tdTomato signal 24h after transfection. Of each well a representative picture with the following settings was made: Exposure: 500ms, Gain: 1.5, Intensity: 30 %.

### Results:

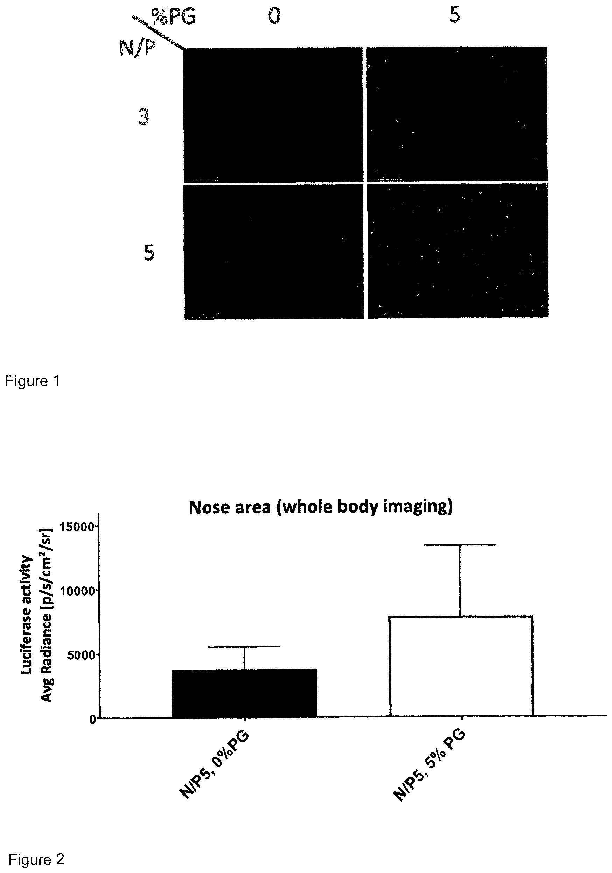

For the treatment of ALI cultures an mRNA encoding for tdTomato was used. Treated wells were analyzed for tdTomato protein 24h after treatment. In Figure 1, exemplary pictures are shown.

As shown in Figure 1, the addition of propylene glycol significantly increases the level of mRNA that is delivered to the cells and translated into protein. This effect can be observed at N/P 3 as well as N/P 5.

### Example II: Nasal application of complexes

### Methods:

Formulation of RNA-Lipid complex:
Complexes were formed as previously described in Example I

### Treatment of animals:

Balb/c mice (Charles River Laboratories) were treated with complexes at an mRNA dose of 10µg/40µL. The solution was applied as one droplet onto the nostrils of the animal during Isoflurane (Isothesia, Henry Shine, Germany) inhalation anesthesia.

### Detection of luciferase level in nose tissue:

5h after application 1.5mg D-Luciferin dissolved in 50µL PBS was applied intranasally (sniffing method) on the nostrils of each animal. Bioluminescence imaging was conducted using an IVIS 100 in vivo imaging system (Perkin Elmer, USA). Immediately after imaging, the animals were sacrificed by cervical dislocation. The pictures were analyzed using Living Image software (Perkin Elmer, USA) via measurement of the radiance in a set region of interest (ROI). The values are shown as average radiance [p/s/cm²/sr].

### Results:

For the treatment of Balb/c mice via sniffing to the nose mRNA encoding for firefly luciferase complexed at N/P 5 was used. Results of the analysis of the nose tissue for luciferase activity 5h after treatment (Figure 2) show that complexes in a 5% propylene glycol solution results in higher reporter protein activity in the nose tissue compared to the same complexes in absence of propylene glycole (0% PG, water).

### Example III: Stabilization of particle size during one freeze-thaw cycle

Methods:
Formulation of particles:
Particles were formed as described in Example I.

Freezing of particle solution:
After formulation, particles were frozen by storing them for at least 16h at -20°C.

Size measurement:
The hydrodynamic diameter of the particles was measured by dynamic light scattering using a ZetaSizer Nano ZS (Malvern instruments). For a correct measurement, the buffer composition used by the software to calculate the solutions viscocity was adjusted to a 1,2-propanediol containing solution, if applicable.

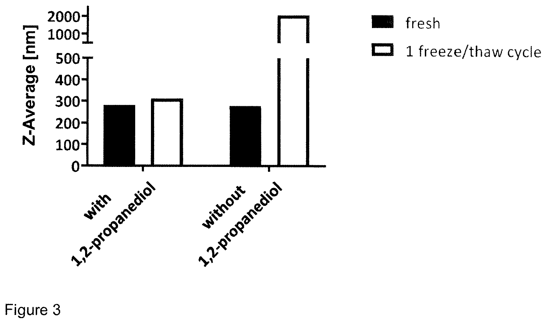

Results:
Particles were formulated. After formulation, the particle size of the fresh particles was measured in a solution with or without 1,2-propanediol. The particles were frozen for 16h at -20°C and thawed by incubation at RT. The particle size in the thawed solutions was measured again. To maintain activity, the deviation of the particle size before freezing and after thawing should be minimal. As shown in Figure 3, particles that were frozen in a solution without 1,2-propanediol significantly increase in size due to aggregation processes during freezing. In the presence of 1,2-propanediol, the particle size remains stable after the freeze thaw cycle.

### Figures:

Figure 1 shows the tdTomato level after transfection with GL67-complexes in different excipient solutions (water (0% propylene glycol) or 5% propylene glycol) at N/P 3 and 5.
Figure 2 shows the luciferase activity in the nose of Balb/c mice 5h after treatment with GL67 complexes in water or in a 5% propylene glycol solution.
Figure 3 shows the particle size before freezing and after thawing in the presence or absence of 1,2-propanediol.

## Claims

1. A composition comprising
(i) particles contained in a liquid phase, wherein the particles comprise RNA and a lipid composition, and wherein the lipid composition comprises:
(i-a) a cholesterol derivative of formula (I) or a salt thereof: 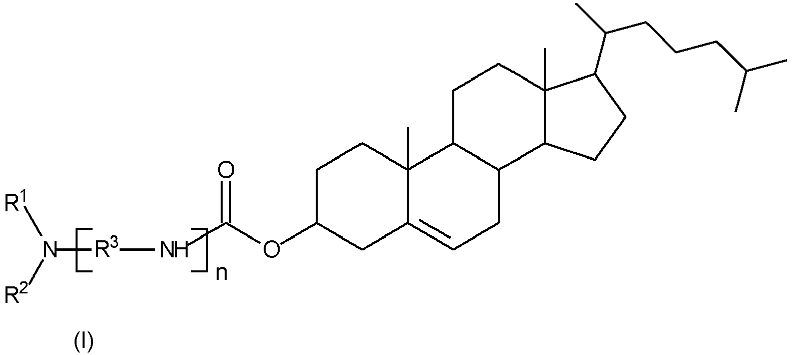 wherein
n is 0 or 1, preferably 0,
R¹ is a group -(CH₂)_{q}-NH₂ or a group -(CH₂)ᵣ-NH-(CH₂)ₛ-NH₂, wherein q, r and s are independently an integer of 2 to 6,
R² is a group -(CH₂)ₜ-NH₂ or a group -(CH₂)ᵤ-NH-(CH₂)_{w}-NH₂, wherein t, u and w are independently an integer of 2 to 6,
R³ is a linear alkanediyl group having 1 to 4 carbon atoms;
(i-b) a phosphoglyceride of formula (II) or a salt thereof: 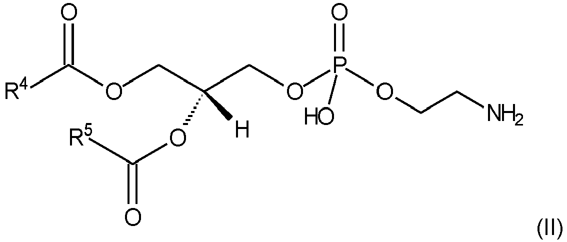 wherein
R⁴ is a linear alkyl group having 10 to 24 carbon atoms or a linear alkenyl group having 1 to 3 double bonds and 10 to 24 carbon atoms;
R⁵ is a linear alkyl group having 10 to 24 carbon atoms or a linear alkenyl group having 1 to 3 double bonds and 10 to 24 carbon atoms; and
(i-c) a pegylated phosphoglyceride of formula (III) or a salt thereof: 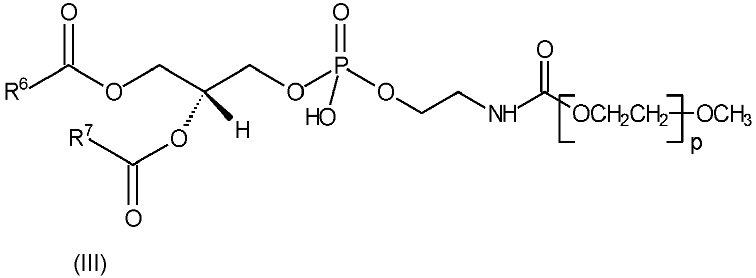 wherein
p is an integer of 5 to 200, preferably 10 to 170 and most preferably 10 to 140
R⁶ is a linear alkyl group having 10 to 20 carbon atoms or a linear alkenyl group having 1 to 3 double bonds and 10 to 20 carbon atoms;
R⁷ is a linear alkyl group having 10 to 20 carbon atoms or a linear alkenyl group having 1 to 3 double bonds and 10 to 20 carbon atoms;
and
(ii) 1,2-propanediol.

2. The composition according to claim 1, wherein the RNA is mRNA.

3. The composition according to claim 1 or 2, wherein the cholesterol derivative of formula (I) is GL67.

4. The composition according to any of claims 1 to 3, wherein the phosphoglyceride of formula (II) is 1,2-dioleoyl-*sn*-glycero-3-phosphoethanolamine.

5. The composition according to any of claims 1 to 4, wherein the pegylated phosphoglyceride of formula (III) is 1,2-dimyristoyl-*sn*-glycero-3-phosphoethanolamine-PEG5000.

6. The composition according to any of claims 1 to 5, wherein the molar ratio of the components (i-a) : (i-b) : (i-c) in the lipid composition is 1 : (0.5 to 5) : (0.01 to 1).

7. The composition according to any of claims 1 to 6, wherein the particles have average particle diameter in the range of 1 to 5000 nm.

8. The composition according to any of claims 1 to 7, wherein the N/P ratio of the number of nitrogen atoms N derived from the cholesterol derivative of formula (I) to the number of phosphate groups P in the RNA is in the range of 1 to 100.

9. The composition according to any of claims 1 to 8, wherein the 1,2-propanediol is contained at a concentration of 0.1 to 50 % w/v, based on the total volume of the composition.

10. The composition according to any of claims 1 to 9, wherein the liquid phase in which the particles are contained comprises water.

11. A process for the preparation of a composition in accordance with any of claims 1 to 10, said process comprising the steps of:
a) dissolving and mixing the components of the lipid composition in an organic solvent, followed by the lyophilization of the lipid composition;
b) rehydrating the lyophilized lipid composition via addition of water;
c) combining the rehydrated lipid composition with an aqueous solution of the RNA to allow particles comprising RNA and the lipid composition to be formed which are contained in a liquid phase; and
d) adding 1,2-propanediol.

12. The composition in accordance with any of claims 1 to 10 for use in the treatment or prevention of a disease via an RNA-based therapy.

13. The composition for use in accordance with claim 12 wherein the disease to be treated or prevented is a lung disease.

14. The composition for use in accordance with claim 12 or 13, wherein the treatment or prevention involves the administration of the composition to or via the respiratory tract, preferably via pulmonary administration or nasal administration.

## Patentansprüche

1. Eine Zusammensetzung, umfassend
(i) in einer Flüssigphase enthaltene Partikel, wobei die Partikel RNA und eine Lipidzusammensetzung umfassen, und wobei die Lipidzusammensetzung umfasst:
(i-a) ein Cholesterinderivat der Formel (I) oder ein Salz davon: 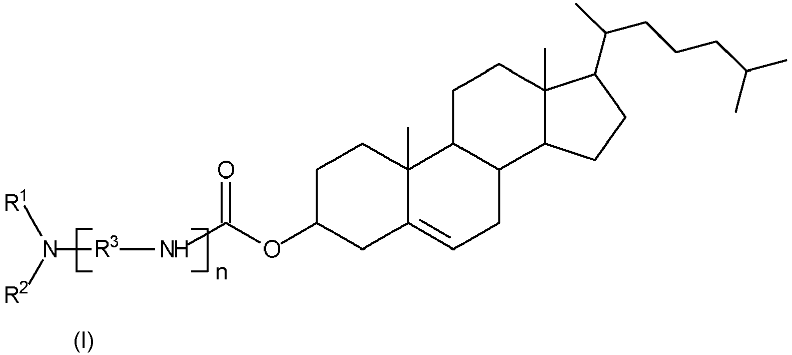 wobei
n für 0 oder 1, vorzugsweise für 0, steht,
R¹ für eine Gruppe -(CH₂)_{q}-NH₂ oder eine Gruppe -(CH₂)ᵣ-NH-(CH₂)ₛ-NH₂ steht, wobei q, r und s unabhängig voneinander für eine ganze Zahl von 2 bis 6 stehen,
R² für eine Gruppe -(CH₂)ₜ-NH₂ oder eine Gruppe -(CH₂)ᵤ-NH-(CH₂)_{w}-NH₂ steht, wobei t, u und w unabhängig voneinander für eine ganze Zahl von 2 bis 6 stehen,
R³ für eine geradkettige Alkandiylgruppe mit 1 bis 4 Kohlenstoffatomen steht;
(i-b) ein Phosphoglycerid der Formel (II) oder ein Salz davon: 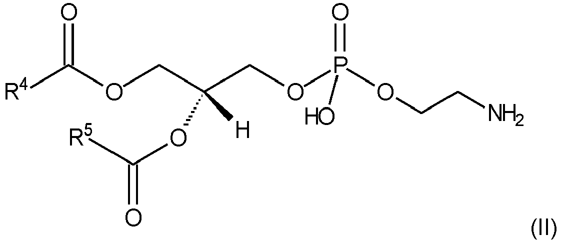 wobei
R⁴ für eine geradkettige Alkylgruppe mit 10 bis 24 Kohlenstoffatomen oder eine geradkettige Alkenylgruppe mit 1 bis 3 Doppelbindungen und 10 bis 24 Kohlenstoffatomen steht;
R⁵ für eine geradkettige Alkylgruppe mit 10 bis 24 Kohlenstoffatomen oder eine geradkettige Alkenylgruppe mit 1 bis 3 Doppelbindungen und 10 bis 24 Kohlenstoffatomen steht; und
(i-c) ein pegyliertes Phosphoglycerid der Formel (III) oder ein Salz davon: 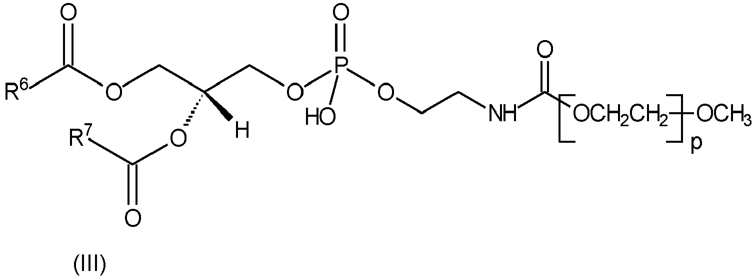 wobei
p für eine ganze Zahl von 5 bis 200, vorzugsweise 10 bis 170 und am meisten bevorzugt 10 bis 140, steht,
R⁶ für eine geradkettige Alkylgruppe mit 10 bis 20 Kohlenstoffatomen oder eine geradkettige Alkenylgruppe mit 1 bis 3 Doppelbindungen und 10 bis 20 Kohlenstoffatomen steht;
R⁷ für eine geradkettige Alkylgruppe mit 10 bis 20 Kohlenstoffatomen oder eine geradkettige Alkenylgruppe mit 1 bis 3 Doppelbindungen und 10 bis 20 Kohlenstoffatomen steht;
und
(ii) 1,2-Propandiol.

2. Die Zusammensetzung gemäß Anspruch 1, wobei die RNA mRNA ist.

3. Die Zusammensetzung gemäß Anspruch 1 oder 2, wobei das Cholesterinderivat der Formel (I) GL67 ist.

4. Die Zusammensetzung gemäß einem der Ansprüche 1 bis 3, wobei das Phosphoglycerid der Formel (II) 1,2-Dioleoyl-*sn*-glycero-3-phosphoethanolamin ist.

5. Die Zusammensetzung gemäß einem der Ansprüche 1 bis 4, wobei das pegylierte Phosphoglycerid der Formel (III) 1,2-Dimyristoyl-*sn*-glycero-3-phosphoethanolamin-PEG5000 ist.

6. Die Zusammensetzung gemäß einem der Ansprüche 1 bis 5, wobei das Molverhältnis der Komponenten (i-a) : (i-b) : (i-c) in der Lipidzusammensetzung 1 : (0,5 bis 5) : (0,01 bis 1) beträgt.

7. Die Zusammensetzung gemäß einem der Ansprüche 1 bis 6, wobei die Partikel einen durchschnittlichen Partikeldurchmesser im Bereich von 1 bis 5000 nm aufweisen.

8. Die Zusammensetzung gemäß einem der Ansprüche 1 bis 7, wobei das N/P-Verhältnis der Anzahl an Stickstoffatomen N, welche von dem Cholesterinderivat der Formel (I) abgeleitet sind, zur Anzahl der Phosphatgruppen P in der RNA im Bereich von 1 bis 100 liegt.

9. Die Zusammensetzung gemäß einem der Ansprüche 1 bis 8, wobei das 1,2-Propandiol in einer Konzentration von 0,1 zu 50 % w/v, bezogen auf das Gesamtvolumen der Zusammensetzung, enthalten ist.

10. Die Zusammensetzung gemäß einem der Ansprüche 1 bis 9, wobei die Flüssigphase, in welcher die Partikel enthalten sind, Wasser umfasst.

11. Ein Verfahren zur Herstellung einer Zusammensetzung gemäß einem der Ansprüche 1 bis 10, wobei das Verfahren die Schritte umfasst:
a) Lösen und Mischen der Komponenten der Lipidzusammensetzung in einem organischen Lösungsmittel, gefolgt von der Lyophilisierung der Lipidzusammensetzung;
b) Rehydrieren der lyophilisierten Lipidzusammensetzung mittels Zugabe von Wasser;
c) Kombinieren der rehydrierten Lipidzusammensetzung mit einer wässrigen Lösung der RNA, um die Bildung von Partikeln zu ermöglichen, die RNA und die Lipidzusammensetzung umfassen und in einer flüssigen Phase enthalten sind; und
d) Zugeben von 1,2-Propandiol.

12. Die Zusammensetzung gemäß einem der Ansprüche 1 bis 10 zur Verwendung bei der Behandlung oder Vorbeugung einer Erkrankung mittels einer RNA-basierten Therapie.

13. Die Zusammensetzung zur Verwendung gemäß Anspruch 12, wobei die zu behandelnde oder zu verhindernde Erkrankung eine Lungenerkrankung ist.

14. Die Zusammensetzung zur Verwendung gemäß Anspruch 12 oder 13, wobei die Behandlung oder Vorbeugung die Verabreichung der Zusammensetzung in die oder über die Atemwege, vorzugsweise mittels pulmonaler Verabreichung oder nasaler Verabreichung, einschließt.

## Revendications

1. Composition comprenant
(i) des particules contenues dans une phase liquide, lesquelles particules comprennent un ARN et une composition lipidique, et laquelle composition lipidique comprend :
(i-a) un dérivé de cholestérol de formule (I) ou un sel de celui-ci : 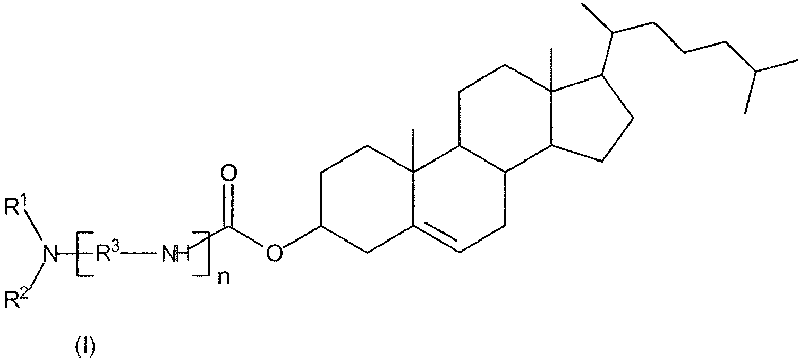 dans laquelle
n vaut 0 ou 1, de préférence 0,
R¹ est un groupe -(CH₂)_{q}-NH₂ ou un groupe -(CH₂)ᵣNH-(CH₂)ₛ-NH₂, où q, r et s sont indépendamment des entiers de 2 à 6,
R² est un groupe -(CH₂)ₜNH₂ ou un groupe -(CH₂)ᵤ-NH-(CH₂)_{w}-NH₂, où t, u et w sont indépendamment des entiers de 2 à 6,
R³ est un groupe alcanediyle ayant 1 à 4 atomes de carbone ;
(i-b) un phosphoglycéride de formule (II) ou un sel de celui-ci : 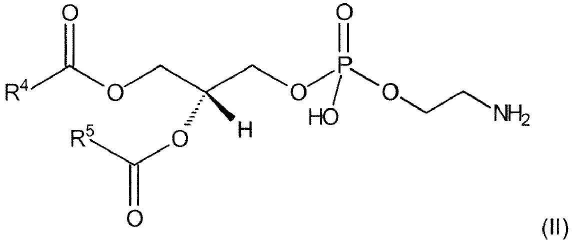 dans laquelle
R⁴ est un groupe alkyle linéaire ayant 10 à 24 atomes de carbone ou un groupe alcényle linéaire ayant 1 à 3 doubles liaisons et 10 à 24 atomes de carbone ;
R⁵ est un groupe alkyle linéaire ayant 10 à 24 atomes de carbone ou un groupe alcényle linéaire ayant 1 à 3 doubles liaisons et 10 à 24 atomes de carbone ;
et
(i-c) un phosphoglycéride PEG-ylé de formule (III) ou un sel de celui-ci : 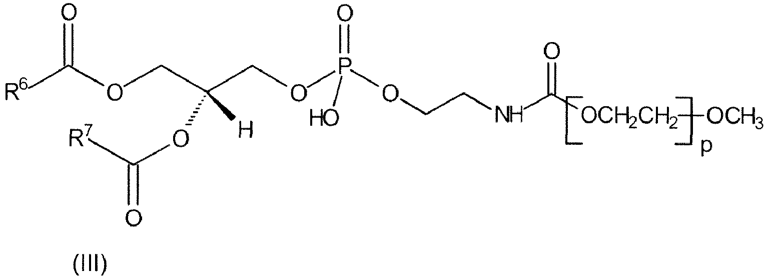 dans laquelle
p est un entier de 5 à 200, de préférence de 10 à 170 et tout spécialement de 10 à 140
R⁶ est un groupe alkyle linéaire ayant 10 à 20 atomes de carbone ou un groupe alcényle linéaire ayant 1 à 3 doubles liaisons et 10 à 20 atomes de carbone ;
R⁷ est un groupe alkyle linéaire ayant 10 à 20 atomes de carbone ou un groupe alcényle linéaire ayant 1 à 3 doubles liaisons et 10 à 20 atomes de carbone ;
et
(ii) du 1,2-propanediol.

2. Composition selon la revendication 1, dans laquelle l'ARN est un ARNm.

3. Composition selon la revendication 1 ou 2, dans laquelle le dérivé de cholestérol de formule (I) est GL67.

4. Composition selon l'une quelconque des revendications 1 à 3, dans laquelle le phosphoglycéride de formule (II) est la 1,2-dioléoyl-*sn*-glycéro-3-phosphoéthanolamine.

5. Composition selon l'une quelconque des revendications 1 à 4, dans laquelle le phosphoglycéride PEG-ylé de formule (III) est la 1,2-dimyristoyl-*sn*-glycéro-3-phosphoéthanolamine-PEG5 000.

6. Composition selon l'une quelconque des revendications 1 à 5, dans laquelle le rapport molaire des composants (i-a)/(i-b)/(i-c) dans la composition lipidique est de 1/(0,5 à 5)/(0,01 à 1).

7. Composition selon l'une quelconque des revendications 1 à 6, dans laquelle les particules ont un diamètre moyen situé dans la plage allant de 1 à 5 000 nm.

8. Composition selon l'une quelconque des revendications 1 à 7, dans laquelle le rapport N/P du nombre d'atomes d'azote N dérivés du dérivé de cholestérol de formule (I) au nombre de groupes phosphate P dans l'ARN est situé dans la plage allant de 1 à 100.

9. Composition selon l'une quelconque des revendications 1 à 8, dans laquelle le 1,2-propanediol est contenu à une concentration de 0,1 à 50 % p/v, basés sur le volume total de la composition.

10. Composition selon l'une quelconque des revendications 1 à 9, dans laquelle la phase liquide dans laquelle les particules sont contenues comprend de l'eau.

11. Procédé pour la préparation d'une composition selon l'une quelconque des revendications 1 à 10, ledit procédé comprenant les étapes de :
a) dissolution et mélange des composants de la composition lipidique dans un solvant organique suivis de la lyophilisation de la composition lipidique ;
b) réhydratation de la composition lipidique lyophilisée par l'addition d'eau ;
c) combinaison de la composition lipidique réhydratée avec une solution aqueuse de l'ARN pour permettre la formation de particules comprenant de l'ARN et la composition lipidique, qui sont contenues dans une phase liquide ; et
d) addition de 1,2-propanediol.

12. Composition selon l'une quelconque des revendications 1 à 10 pour une utilisation dans le traitement ou la prévention d'une maladie par une thérapie basée sur l'ARN.

13. Composition pour une utilisation selon la revendication 12, dans laquelle la maladie devant être traitée ou prévenue est une maladie pulmonaire.

14. Composition pour une utilisation selon la revendication 12 ou 13, dans laquelle le traitement ou la prévention met en jeu l'administration de la composition aux voies respiratoires ou via les voies respiratoires, de préférence via une administration pulmonaire ou une administration nasale.
